# EUROPEAN PATENT SPECIFICATION

(11) **EP 4 658 251 B1**
(45) Date of publication and mention of the grant of the patent: **18.11.2026**
(21) Application number: 24703294.9
(22) Date of filing: 30.01.2024
(51) Int. Cl.: A61K 9/1276, A61K 31/045, A61K 31/047, A61K 31/19, A61P 35/00, A23L 33/10

(54) **COMPOSITION AND METHOD OF PREPARATION OF BIOACTIVE TRITERPENOID MILK FAT GLOBULE FORMULATIONS**
ZUSAMMENSETZUNG UND VERFAHREN ZUR HERSTELLUNG BIOAKTIVER TRITERPENOID-MILCHFETTKÜGELCHENFORMULIERUNGEN
COMPOSITION ET PROCÉDÉ DE PRÉPARATION DE FORMULATIONS DE GLOBULES DE LA MATIÈRE GRASSE LAITIÈRE À BASE DE TRITERPÉNOÏDES BIOACTIFS

(30) Priority: 30.01.2023 NL 2034054
(43) Date of publication of application: 10.12.2025
(73) Proprietor: Efnatics B.V., 2312 ND Leiden (NL)
(72) Inventor: KESSLER, Johan Herman, 2312 ND LEIDEN (NL)
(74) Representative: Herrero & Asociados, S.L.
(86) International application number: PCT/EP2024/052217
(87) International publication number: WO 2024/160809

(56) References cited:
- US-A- 5 298 246
- US-A1- 2014 105 875
- US-A1- 2016 000 710
- ANKIT SANEJA ET AL: "Therapeutic applications of betulinic acid nanoformulations", ANNALS OF THE NEW YORK ACADEMY OF SCIENCES, NEW YORK ACADEMY OF SCIENCES, US, vol. 1421, no. 1, 29 January 2018 (2018-01-29), pages 5 - 18, XP071410440, ISSN: 0077-8923, DOI: 10.1111/NYAS.13570

## Description

### Technical field

The present invention relates to a specific formulation for bioactive pentacyclic triterpenoids, such as betulinic acid and betulin, in particular for the use as pharmaceutical or nutraceutical, more particularly for use of the treatment or prevention of cancer.

### 1. Background of the invention

Plant derived molecules are at the base of many important chemotherapeutical drugs for cancer and other diseases, either directly or as lead compound [1,2], and constitute great potential for new drugs [3]. Pentacyclic triterpenoids are one of the largest class of natural plant secondary metabolites with profound beneficial biological effects and pharmacological potential. These compounds own a 30-carbon skeleton comprising five ring structures, either five six-membered rings (ursane- and oleanane-subtypes) or four six-membered rings with one five-membered ring (lupane subtype).

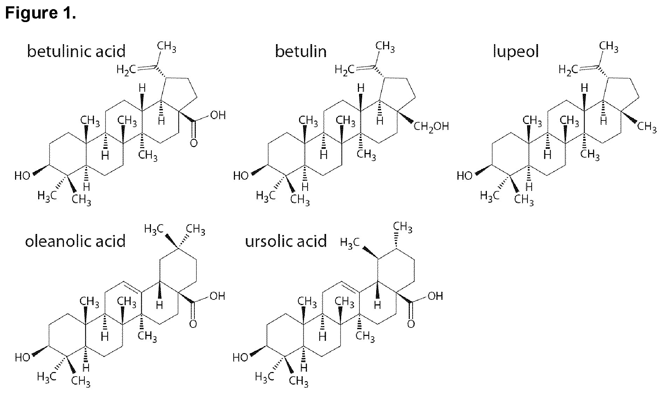

The phytochemical compound betulinic acid is a member of the lupane-type pentacyclic triterpenoids with an established anti-cancer activity demonstrated in vitro and in animal experiments. Betulin, another biological active member of this class, is a direct analogue of betulinic acid with the sole difference that it lacks the carboxyl group at the C-28 (carbon) atom of the lupane skeleton. Lupeol is a third member of the pentacyclic triterpenoids with a lupane scaffold and also has profound anticancer and anti-inflammatory biological effects [4]. Also oleanolic acid and ursolic acid, two isomeric analogue pentacyclic triterpenoids of the oleanane- and the ursane-type, respectively, own established favorable biological effects, especially anti-cancer activity [5]. The chemical structures of these bioactive pentacyclic triterpenoids are shown in Fig. 1.

Betulinic acid, betulin, lupeol, oleanolic acid and ursolic acid are all abundantly available in many plant species. For instance the outer bark of the white-barked birch trees (betula serie albae) contains abundant quantities of betulin, up to 30% of dry weight bark [6], and betulinic acid at approximately one tenth of that quantity [7]. In particular betulinic acid is known for its broad activity against all prevalent cancer types in vitro [8] and inhibits tumor growth of predominant cancer types preclinically in vivo [9]. Other activities of the compound are established as well, like its anti-inflammatory effect, its anti-HIV effect, and beneficial effects against diabetes type 2 [10-12].

Importantly, cytotoxicity of betulinic acid for non-cancerous healthy cells was shown to be absent in vitro. Also at concentrations that are effectively limiting cancer growth in animal experiments, up to 500 mg per kg bodyweight, no signs of toxicity were observed [9,13], indicating its favorable therapeutic index for clinical usage.

Mechanistically, the anti-cancer capacity of betulinic acid has been pinpointed to diverse effects, pathways and molecules, which are partly intertwined. The selective cytotoxicity of betulinic acid for cancer cells can be explained by its specific mechanistic effect on metabolic pathways which are adapted in dividing cancer cells when compared to healthy cells, rendering cancer cells vulnerable and these pathways a selective target for drug intervention [14]. For instance, betulinic acid was shown to inhibit the activity of stearoyl-CoA-desaturase (SCD-1), an enzyme of which the correct function in cellular lipid metabolism is crucial for cancer cell divisions and thus tumor growth [15]. Likewise, the effect of betulinic acid on glucose metabolism in cancer cells [16], which is changed as compared to healthy cells (the so-called Warburg effect [17]), likely contributes to the selectivity of the compound for cancer cells and also its polypharmacology in a broader sense [18].

Taken together, these bioactive pentacyclic triterpenoids and in particular betulinic acid, are attractive drug candidates against the above-mentioned conditions. Due to its lack of toxicity for healthy tissues, its preclinical established synergistic and priming effect in conjunction with standard care cancer drugs [19-23] and its proposed role in prevention of cancer [24-26] and other conditions like e.g. hepatic toxicity [27], diabetes type 2 [28] and diabetes-related conditions [29,30], particularly betulinic acid, next to being a candidate adjuvant cancer drug, also constitutes a promising nutraceutical or food supplement.

However, despite their promising activity profiles and absent toxicity, the preclinical development of these pentacyclic triterpenoids aiming at clinical use has been strongly hindered by important disadvantageous characteristics.

Firstly, the bioactive triterpenoids addressed herein are extremely hydrophobic with consequently poor aqueous solubility, which makes it a great challenge to render these compounds sufficiently bioavailable.

Secondly, the molar concentration required for desired strong biological effects is rather high (as compared to e.g. some established cancer drugs), e.g. in the range of 10 - 20 µM in vitro, in studies with cancer cell lines [8], underlining the importance to devise formulations that not only enable a good solubility of the triterpenoids, but that also incorporate these compounds at high payload [31].

To address the poor aqueous solubility of the triterpenoids of interest there are basically two strategies available in the art, elaborated in a multitude of studies thus far. Either semisynthetic derivatives with a higher solubility than their natural analogue triterpenes have been pursued [11,32-35] or pharmaceutical drug carrier and delivery systems were developed aiming at high solubility and efficient bioavailability of the compounds.

However, despite the identification of several interesting derivatives, none has made it to clinical use as a pharmaceutical or nutraceutical. Therefore, considering the only moderately improved aqueous solubility of the triterpene derivatives of the prior art, if improved at all, together with the abundant availability of the natural compounds in plant species (e.g. in birch trees, for betulinic acid and betulin), the natural pentacyclic triterpenoid compounds must still be considered as the prime candidates to develop for human use.

### 1.1 Desirable features of triterpenoids formulations

A prerequisite for the use of the natural pentacyclic triterpenoids, in particular betulinic acid and betulin, is the development of a drug carrier system enabling their efficient solubilization. Importantly, any formulation addressing the two disadvantageous characteristics of the pentacyclic triterpenoids herein addressed - being their poor solubility and the required high in vivo bioavailable concentration - by efficiently incorporating the triterpenoids at high concentration, should apart from having a high payload of the compound also enable their efficient intestinal delivery, uptake and absorption after oral administration [36,37].

Oral administration is the preferred route for the triterpenoid compounds when used as adjuvant drug in combination treatments of cancer [19,20], and obviously so when used as dietary food supplement or nutraceutical for anti-cancer chemoprevention [25], for chemoprevention of other disease states and for accomplishing other beneficial health effects.

Thus, the drug carrier ideally meets a threefold-demand, being firstly the efficient solubilization at high drug loading capacity of the triterpenoid in the carrier system, where drug loading capacity can be defined as the percentage of mass of the drug carrier that is due to the encapsulated drug. Secondly the carrier system should warrant an efficient in vivo intestinal absorption of the entrapped triterpenoid compound. As a third demand, the triterpenoid delivery system should itself preferably lack any excipient that potentially confers disadvantageous toxic side effects, especially when applied pharmaceutically in conjunction with standard care cancer chemotherapy or as nutraceutical.

Taken together, to develop such a delivery system is a great challenge and the current problem to be solved for the application in human of these promising pentacyclic triterpenoids.

### 1.2 Formulations of triterpenoids in the art, as elaborated for betulinic acid

The field of drug delivery has been rapidly evolving the last decade, in particular various organic nanoparticle-based systems have been developed aiming at the solubilization of otherwise insufficiently bioavailable pharmaceuticals. A variety of these nanoparticle-based drug carriers have been explored for their capacity to solubilize and transport the pentacyclic triterpenoids betulinic acid, betulin, lupeol, oleanolic acid and ursolic acid or their semisynthetic derivatives. In different model systems, both in vitro and in vivo, such new formulations incorporating prominent triterpenes have been tested [38,39]. Especially for betulinic acid various delivery systems have been devised and tested of which the most important prior art literature is discussed herein, and summarized in Table 1.

The nanoscale delivery systems exploited for betulinic acid include among others polymeric nanoparticles on carbohydrate-base, like cyclodextrins [40] and chitosan [41], dendrimers [41], poly(lactic-co-glycolic acid)(PLGA) nanoparticles [42,43], and betulinic acid-containing PLGA-nanoparticles co-polymerized with polyethylene glycol (PEG) [44].

Lipid-based nanoparticles is another broad class of carriers that are applied for the delivery of lipophilic anti-cancer compounds [45]. Lipid carriers commonly have a reasonably good loading capacity. Their scaled up production from natural sources is - depending on the precise lipidic carrier type - often possible for reasonable costs. Moreover, lipid-based formulations are in general considered beneficial for intestinal absorption of the lipophilic active pharmaceutical ingredient contained in the carrier [46]. Within this category, betulinic acid has been tested, in vitro and in vivo, encapsulated in liposomes as carrier [9,47] and in oil-in-water nanoemulsion [48].

Despite the many efforts in the betulinic acid drug delivery field, thus far none of the reported formulations stand out as favorable. In general, liposomes, of which a plethora of types exist, are the lipidic (nano)particles that are used most often as drug carrier [49]. Liposomes have good possibilities as drug carrier, but disadvantages related to liposomes exist as well [50], such as their moderate loading capacity for hydrophobic compounds and the high production costs of advanced types of liposomes.

For the delivery of bioactive triterpenoids, administered in a pharmaceutical or nutraceutical context, a carrier ideally should entrap a sufficiently high quantity of the active compound and the formulation should have additional favorable characteristics when applied orally. Liposomes fulfill these prerequisites likely insufficiently well, as studies have shown. For instance, the maximal loading capacity of betulinic acid in big liposomes appeared to be 5 mg betulinic acid per ml liposomes in a study where betulinic acid was tested in vivo [9].

This would indicate, as extrapolated from the weekly orally administered betulinic acid dose of 150 mg/kg bodyweight required for the observed anti-tumor effects in mice [9], that an individual of 80 kg needs a dose of 12 gr betulinic acid weekly. Thus requiring a weekly consumption of 2.4 Itr of such a liposomal formulation (at 5 mg/ml betulinic acid) for a human. Such an intake is impossible. Patient (or client) compliance will be very low and, moreover, such extremely high daily quantities of lipidic liposomal excipients are not healthy. Proliposomes, which are recently developed carbohydrate carriers coated with phospholipids in powder form (administered e.g. in pills) that upon addition of water reconstitute into liposomes [51], will have the same problem: an excessive quantity of excipient intake is required to reach a sufficient bioavailability of the active triterpenoid.

Cancer preventive effects (i.e. chemoprevention) instead of therapeutic effects of betulinic acid against cancer may be reached at somewhat lower in vivo concentrations, and possibly the extrapolation from mice studies to human use can be adjusted to a slightly lower human dose per kg bodyweight, because of the high metabolism rate in mice [52]. However, an effective oral use of pentacyclic triterpenoid formulations for human health promotion will likely heavily rely on the payload of the active pharmaceutical ingredient in the formulation.

The first focus in developing a suitable formulation for the oral administration of triterpenoids is therefore its high payload, preferably at concentrations far exceeding the aforementioned 5 mg active ingredient per ml formulation, which is the highest liposomal payload reached in the prior art. This is the main problem to be solved in this field.

In addition, any oral formulation should guarantee a good intestinal absorption of the triterpenoid and the excipients of the delivery system should lack any toxicity, and should preferably be allowed as food additives, as listed e.g. in the 'generally recognized as safe' (GRAS) FDA category, or even better constitute themselves natural food or elements thereof.

Liposomal formulations are in general not considered as a drug carrier of choice for oral delivery [53,54]. Liposomal and other triterpenoid formulations developed thus far all have weaknesses, as mentioned before and elaborated for the case of betulinic acid, with a summary of the most prominent literature, in Table 1. Therefore, an ongoing need exists for a suitable formulation for bioactive triterpenoids allowing their oral administration in a feasible dosage form and at a dosage of the active compound effectively achieving high bioavailability.

It is an objective of the present invention to overcome one or more of the above-mentioned problems and in addition possible related problems.

### 2. Summary of the invention

In an aspect, the present invention provides a formulation composition of a microparticle carrier system for the oral delivery at high dose of extremely hydrophobic bioactive pentacyclic triterpenoids such as betulinic acid, betulin, lupeol, oleanolic acid and ursolic acid. The present invention also provides a method of preparation thereof.

The triterpenoid formulations of the invention are preferably composed of natural (food) components as primary excipients, in particular milk fat globules, and can be applied as nutraceutical or pharmaceutical without adverse side-effects for the improvement of general health and for treatment, chemoprevention or amelioration of prevalent disease states like cancer, cardiovascular disease and diabetes type 2.

High-energy solubilization and emulsification methods, preferably high shear mixing in combination with sonication, can be used in the present invention to solubilize and load the triterpenoids at high concentration into milk fat globules, which preferably is the primary excipient of the microparticle drug carrier system.

### 2.1 The need in the field to be addressed

The bioactive pentacyclic triterpenoids of the invention, of which the chemical structures are depicted in Fig. 1, in particular betulinic acid and betulin, and even more in particular betulinic acid, have great potential as drug or nutraceutical compound for the treatment, chemoprevention and amelioration of prevalent and serious disease states, as is wellsupported by a multitude of preclinical in vitro and animal studies [55]. For human use, however, important properties to be met are still lacking in the field, which is the primary cause of a predominantly absence of clinical testing of the bioactive triterpenoids.

Firstly, the required pharmacologically active dose of these triterpenoids typically is relatively high (e.g. as compared to most established cancer drugs). As being extrapolated from animal studies, a high dose is useful for efficacious triterpenoid bioactivity to occur [9]. Such a high dose is possible to administer by the absence of toxicity for healthy cells and tissues at such a dose [9]. Therefore, most importantly, for clinical use the formulation composition and its manufacturing process should enable efficient solubilization and should achieve very high incorporation concentrations of the triterpenoid in the drug delivery formulation.

Ideally, in addition, the triterpenoid carrier should be efficiently taken up in the gastrointestinal tract and absorbed after oral administration, rendering the triterpenoids bioavailable at high in vivo dose.

Moreover, as important third desired aspect, the excipients of the drug carrier system should ideally be biocompatible, preferably constituting food-grade components and be without any harmful effects at necessarily high administered quantities, and more preferably, the excipients confer additional beneficial health effects themselves.

Finally, high stability of the formulation and its cost-effective production are highly desired beneficial properties of the preferred formulation.

The invention herein disclosed is the result of a comprehensive study to discover a solubilization and carrier system fulfilling all of these aforementioned key points, thereby furnishing a solution to the need in the field.

This study tested a great variety of candidate drug delivery compositions and their possible formulation methods for the pentacyclic triterpenoids addressed herein, in particular betulinic acid. Next to liposomes, being to date a highly-established drug carrier platform for especially hydrophilic but also lipophilic compounds, various other organic, especially lipid-based, carrier systems were analyzed. Moreover, many different methods for the successful solubilization, assembly and incorporation of the triterpenoids into these candidate drug carrier systems were analyzed.

### 3. Brief description of the invention

### 3.1 Drug delivery composition of triterpenoids assembled with milk fat globules

The present invention discloses a microparticle formulation which is composed of the bioactive pentacyclic triterpenoids of interest in conjunction with milk fat globules, and preferably assembled using high-energy emulsification methods, in particular sonication. The formulation of the invention is an extremely favorable composition for high-dose solubilization and encapsulation of these bioactive pentacyclic triterpenoids and their subsequent non-toxic oral delivery resulting in anticipated high bioavailability. The triterpenoids are preferably either betulinic acid or betuline, and in the most preferred embodiment betulinic acid.

The composition of lipophilic pentacyclic triterpenoids solubilized in and assembled together with milk fat globules appears to be in all aforementioned desired aspects a favorable drug carrier and delivery system.

Notably, the high payload of the triterpenoids in this delivery system is the result of the joint specific properties of the bioactive triterpenoid compounds on the one hand, and those of the milk fat globules on the other hand. Together, this set of combined properties enable the efficient solubilization at a high level of loading of the triterpenoids in the milk fat globule-based triterpenoid carrier.

### 3.2 Overview of milk components and the structure of milk fat globules

Milk is the evolutionary developed unique source of energy, structural building molecules and protection for neonates. Animal milk, especially of bovine source, and thereof derived dairy products are worldwide a common food. Basically, milk is a colloidal multiphase polydisperse system containing protein particles and lipid vesicle structures. The primary proteins contained in milk are caseins, organized in micelle particles [56,57], and whey proteins [58]. With respect to the milk-contained lipids, milk can be regarded as an oil-in-water macroemulsion.

Milk contains, before any processing, between 2% and 5% lipids [59]. These lipids are organized in milk fat globules, which have a sophisticated structure that only exists in milk. The size of the milk fat globules in raw milk is between 0.1 and 15 µm in diameter [60]. The milk fat globule consists of a core of triglycerides contained by a three-layer lipid membrane, which is assembled during secretion of the milk fat globules from epithelial cells of the mammary gland [61].

In greater detail, the generally supported physiological process is that first the core of triglycerides is coated in the cytoplasm with a monolayer membrane of polar lipids and proteins. Subsequently during secretion of the milk fat globules, a bilayer membrane derived from the secreting epithelial cells is added, which contains glycosylated and non-glycosylated proteins, glycerophospholipids and sphingolipids, enzymes and cholesterol. An interstitial layer of proteins is formed in addition between the monolayer and bilayer lipid membranes during secretion. Together this 10 - 50 nm thick multilayer envelope constitutes the tri-layered milk fat globule membrane (MFGM) [60].

### 3.3 Emulsifier function of components in the milk fat globule membrane

Importantly, the amphipathic character of the polar lipids in the MFGM together with the proteins in the membrane are crucial to prevent the coalescence of the triglycerides in the core of milk fat globules, thereby assuring the oil-in-water emulsion character of milk [62]. Thus the membrane components function as crucial emulsifiers to keep the milk fat globules in emulsion, whereby a differential role exists for the polar lipids and the proteins in the MFGM [63].

Homogenization, which is the process commonly effectuated on industrially processed consumer milk from bovine source to increase its fat stability, profoundly reduces the average size of the milk fat globules and also increases their emulsification properties by inducing adherence of casein micelles to the milk fat globule membrane [64-67].

### 3.4 Aspects of the triterpenoid - milk fat globule formulation

The present invention discloses a solubilization and carrier system of the triterpenoids of interest in a microparticle (alternatively called, micro vesicle) composition completely united with and integrated into milk fat globules.

The triterpenoid formulations of the invention possess a lipid vesicle nature which may be contained in the preferred embodiment in an emulsion with high viscosity.

In addition, the triterpenoid - milk fat globule compositions of the invention can also exist in, or can be processed into, various adapted forms, like emulsions further diluted in e.g. dairybased beverages, ointments or creams adapted for topical application or in powder-form after spray-drying, freeze-drying or similar techniques, which powder can then be used in e.g. food supplements or beverages.

Depending on the initial dairy source of the milk fat globules used for its preparation, i.e. either raw milk or one of several possible post-processed dairy products, for instance homogenized milk, the composition of the invention may contain a certain quantity of additional milk-derived components. In particular casein micelles and/or whey proteins can be present to a certain quantity, or these milk components may be fully or almost fully absent in the composition of the invention.

In some embodiments of the invention these additional milk-derived components may be deliberately added to the milk fat globules used for the preparation of the triterpenoid composition, as they may confer beneficial properties. For instance, casein micelles that adhere to milk fat globules may even further improve the emulsifying properties of the milk fat globule membrane, likewise whey proteins may help to further improve stability of emulsions [68].

### 3.5 Efficient drugloading in the triterpenoid - milk fat globule formulation as compared to nanocarrier systems

The development of drug delivery systems has expanded enormously the last decades, and has radically changed this field. In particular nanosized colloidal carriers, either lipid- or polymer-based, have attracted much attention and have been devised in many different types. Nanocarriers, defined as being sized <100 nm in diameter, but particles up to several 100 nm (even 500 nm) are often coined nano-sized as well [69], have several favorable features as compared to micro-sized delivery systems.

The high surface area to volume ratio of nanocarriers promotes their solubility, bioavailability [65], stability and the small size help to prevent their clearance through immune mechanisms by macrophages of the reticuloendothelial system [70]. As such nanosized particles can show prolonged circulation times in cancer, and - depending on the tumor microenvironment - a passive targeting mechanism known as the enhanced permeation and retention (EPR) effect can advantageously give rise to higher deposition of the carrier-contained drugs in tumors [71].

Nanocarriers have been developed in many different forms and can be classified according to the source of their excipients (natural or synthetic), the excipients being organic or inorganic (the latter e.g. gold nanoparticles for diagnostics), the base of the chemical matrix (lipid-based or polymeric) and their physicochemical particle composition (solid particles or core-shell like vesicles with an aqueous core) [71-73].

In general and as appreciated by the person skilled in the art, for strongly hydrophobic active pharmaceutical ingredients, like pentacyclic triterpenoids, a lipid-based composition is logical to envision as suitable drug (nano)carrier. However, also various polymer-based (nano)carriers have the capacity to harbor hydrophobic compounds [74].

Lipid-based colloidal nanocarriers can be classified in (1) liposomes, (2) solid lipid nanoparticles (SLN), (3) nanostructured lipid carriers (NLC) and (4) nanoemulsions [74,75]. Historically, liposomes, being carriers of the core-shell lipid-based type (i.e. an aqueous core contained in one or more lipid bilayers), are longest studied in the field since their invention in the 1960s. Nano-sized liposomes can be categorized among the nanocarriers, but (much) larger liposomes exist as well (up to >10 um).

In the mid-1990s solid lipid nanoparticles (SLN) and somewhat later the nanostructured lipid carriers (NLC) were developed to overcome several disadvantages of liposomes [74]. SLN have a hydrophobic core of solid lipids, e.g. triglycerides, waxes, or fatty acids, stabilized with emulsifiers, like a phospholipid monolayer and/or Tween or bile salts, which function as coating and surfactant. As a next step, NLC were developed by replacing a part of the solid lipid core of the SLN by liquid lipids, which creates space to further enhance drug loading of hydrophobic active pharmaceutical ingredients (API) and also improves long term stability by preventing API repulsion from the lipid core.

Nanoemulsions of the oil-in-water (O/W) type are quite similar to SLN and NLC but consist fully of liquid lipids, surrounded with an emulsifying monolayer of phospholipids. With respect to drug loading characteristics, liposomes are first of all suited for the transport of hydrophilic API (in their aqueous core) and less efficiently liposomes can also harbor hydrophobic API in their lipid bilayer [74]. The characteristics of SLN, NLC and O/W nanoemulsions render these solely suited for transport of lipophilic bioactive compounds.

This strong emphasis in the drug delivery field on small nano-sized drug carriers during the last two decades can be considered as overhyped to a certain extent [76-78], especially so because micrometer-sized delivery systems in certain respects have advantages as well, depending on the purpose of use. Importantly, the loading capacity of nano-sized particles is considerably lower than the capacity of larger carriers, like large liposomes or (macro)emulsions, because of the small particle volumes [79].

Indeed, with respect to transport of triterpenoids, it was shown that betulinic acid is incorporated at a much higher payload into large liposomes as compared to small nanosized liposomes [9]. The most important betulinic acid formulations published in the literature are summarized in Table 1.

The contemporary biased emphasis on nanosized delivery systems may therefore preclude the discovery of an optimal drug formulation system for the (oral) delivery of pentacyclic triterpenoids. Restricted availability of technological expertise in companies and academic research groups is known to create another bias impeding the discovery of optimal formulation approaches [80].

The invention disclosed herein is the result of an in-depth analysis of the formulation problem to be solved, particularly with respect to the chemical features of the triterpenoids of interest, in combination with a great empirical research effort.

The milk fat globule - triterpenoids drug carriers of the invention are capable to harbor a very high payload of the triterpenoid. This can be explained by the unique combination of properties of the milk fat globule as excipient, on the one hand, and those of the triterpenoids on the other hand, most preferably the triterpenoids are betulinic acid or betulin.

### As shown in the experimental section:

- Betulinic acid and betulin can be efficiently solubilized and incorporated in the milk fat globule - betulinic acid (or betulin) formulation, by using sonication techniques as described and shown in Examples 1 and 2.
- The milk fat globule - betulinic acid (or betulin) formulation of the invention contains a high payload of betulinic acid (or betulin), as shown in Example 2.
- The incorporation of betulinic acid (or betulin) changes the characteristics of the milk fat globule compositions, as shown in Examples 3 and 4.
- In addition, the milk fat globule - betulinic acid formulation has a high stability, as is shown by the data in Example 5.

### 3.6 Features of triterpenoids and milk fat globules that jointly promote high level drug loading

The special characteristics of milk fat globules on the one hand and the bioactive pentacyclic triterpenoids of the invention on the other hand, preferably betulinic acid and betulin, are jointly underlying the favorable properties of the milk fat globules - triterpenoid formulations. In general, milk fat globules are known to be equipped to contain lipophilic bioactives [81-83].

It appears, as disclosed by the current invention, that pentacyclic triterpenoid drug loading is extremely efficient in milk fat globules, particularly when performed by appropriate loading methods.

The relatively large size of (bovine) milk fat globules (on average 4 µm, but after possible preceding homogenization procedures the average size may be smaller) provides a relatively big volume content for lipophilic drug loading in the triglyceride core. In addition, the size and composition of the milk fat globule membrane, being a complex tri-layer membrane composed of different phospholipids and proteins with exquisitely good emulsifier properties, render the membrane equally well suited to harbor high quantities of hydrophobic bioactive ingredients.

The unexpectedly efficient and high triterpenoid-loading capacity of the compositions of the invention reveals that the milk fat globule properties perfectly match with the properties of the pentacyclic triterpenoids. It can be theorized and is highly likely that several special features of the triterpenoids, preferably betulinic acid or betulin, contribute to the apparently efficient physicochemical interaction between milk fat globules and triterpenoids, as detailed herein.

First, it is known that lupane-type pentacyclic triterpenoids, as shown in the literature with betulinic acid and lupeol, are miscible with the main structural outer leaflet membrane phospholipids (glycerophosphatidylcholine and sphingomyelin) at a range of molar ratios [84]. Secondly, and in line with this finding, betulinic acid is known to function like cholesterol as structural element in lipid bilayers of liposomes [9] and there are indeed strong indications of direct interactions between phospholipids and pentacyclic triterpenoids [84-86].

Thirdly, betulinic acid can be solubilized in triglyceride-containing nanoemulsions [48].

Thus, these characteristics of the pentacyclic triterpenoids, in particular betulinic acid, are highly likely underlying the efficient assembly of the milk fat globule-triterpenoid particles and the incorporation of betulinic acid in the milk fat globules, located both in the membrane and in the core, at high payload.

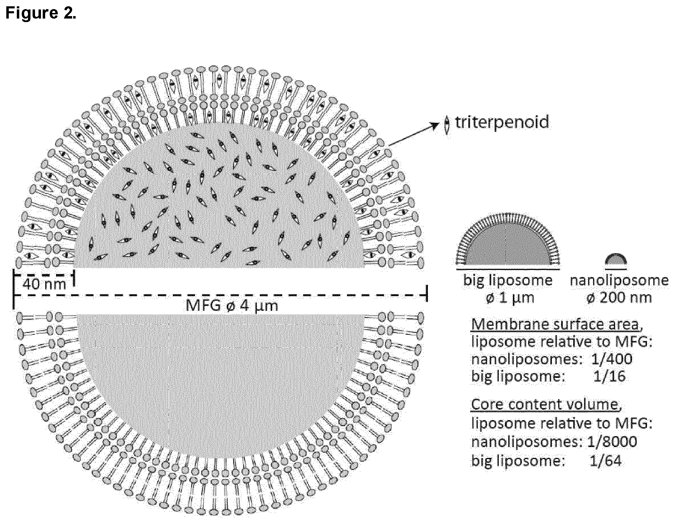

The triterpenoid drug loading in the milk fat globule microvesicle structures can thus take place both in the lipid core and the trilayer membrane, as illustrated in Fig. 2.

Importantly, and notwithstanding certain advantages of nanosized carrier systems (as discussed before), the prime important requisite for efficacious oral delivery of triterpenoids with high bioavailability is better addressed by the micro-sized milk fat globule carrier system. Because, as illustrated in Fig. 2, a great difference exists between the loading capacity of milk fat globules for hydrophobic compounds on the one hand and the loading capacity of nanosized carrier systems, especially (nanosized) liposomes with their aqueous core which cannot harbor hydrophobic bioactive compounds, on the other hand.

### 3.7 Additional advantageous features of triterpenoid-milk fat globule compositions

Apart from optimal drug loading characteristics the milk fat globules have additional features favorable for oral administration of pharmaceuticals and nutraceuticals.

The bioavailability of triterpenoids after their oral administration when contained in a milk fat globule delivery system is expected to be good (shown in Table 1). In general, milk and its components are increasingly considered as excipients for drug delivery systems [87]. Although the precise digestion of milk fat globules in the gastrointestinal tract has not yet been elucidated completely, it is well known that all nutritional components of milk, including the milk fat globules, are exquisitely well digested and absorbed after oral intake [88].

As appreciated by the person skilled in the art, any bioactive compound incorporated in milk fat globules will be released, absorbed and become bioavailable only after the intestinal digestion and uptake of the milk fat globules. Strong indications exist that milk fat globule membrane components can enhance digestion of triglycerides [89] and bioavailability of bioactive compounds [90].

Moreover, the formulations of the invention were found to be stable, which is in line with the literature, because milk fat globule membrane material has the ability to stabilize emulsions [91].

Apart from the advantageous properties of milk fat globule membrane components in digestion and stability, it has been broadly demonstrated that milk fat globule membrane components (the polar lipids, proteins and glycoproteins) themselves also confer beneficial health effects [92]. Obviously, as being a natural food component, milk fat globules will lack any adverse reactions when used as carrier system. Finally, being based on common broadly available milk components, the milk fat globule-triterpenoid formulations of the invention can be manufactured at relatively low-cost basis.

### 4. Detailed description of the invention

The present invention relates to a composition comprising (at least one) triterpenoid compound, preferably pentacyclic triterpenoid compound, wherein the pentacyclic triterpenoid compound is incorporated into milk fat globules or into lipid vesicles having a membrane, wherein the membrane is a tri-layer comprising
- a phospholipid monolayer; and
- a phospholipid bilayer.

Both the triglyceride core and the tri-layered membrane of the milk fat globule or lipid vesicle can serve as lipidic compartments capable to assemble together with the bioactive pentacyclic triterpenoid(s) and to incorporate these.

The milk fat globules or lipid vesicles are surrounded by a tri-layered membrane, wherein the trilayer thus comprises:
- a monolayer of phospholipids, i.e. a lipid monolayer, with the composition typically occurring in the milk fat globule tri-layer membrane; and/or
- a bilayer of phospholipids, i.e. a lipid bilayer.

In an embodiment, one pentacyclic triterpenoid compound, or a combination of two or more different pentacyclic triterpenoid compounds, are comprised in a plurality of milk fat globules.

The (spherical) lipid vesicle or globule according to the present invention has a core comprising triglycerides. The core can be seen as the part of the lipid vesicle or globule that is encapsulated or surrounded by the (outer) tri-layer or tri-layered membrane. The core may comprise for example at least 5 wt.% triglycerides with respect to the weight of the lipid vesicle or globule.

Accordingly, the core may be surrounded or encapsulated by an (inner) monolayer which comprises (polar) phospholipids and/or proteins; and/or an (outer) bilayer of (polar) phospholipids and/or proteins. The proteins may in particular be partially glycosylated and partially non-glycosylated, and/or the lipids may be glycerophospholipids and/or sphingolipids. The monolayer and/or the bilayer may also comprise cholesterol, such as at least 1 wt.% cholesterol with respect to the weight of the particle or globule.

Additionally, an interstitial layer of proteins may be present between the monolayer and the bilayer. The (spherical) tri-layered membrane, i.e. the monolayer, bilayer and optional interstitial layer may be 10 - 50 nm thick. This can be seen as the lipid vesicle or globule's envelope or membrane.

The lipid vesicle or globule of the present invention preferably is a milk fat globule. Accordingly, the present invention preferably relates to a milk fat globule comprising at least one triterpenoid compound.

Thus the invention fundamentally relates to a composition comprising a pentacyclic triterpenoid compound, wherein the pentacyclic triterpenoid compound is incorporated into a milk fat globule or into lipid vesicles having a membrane, wherein the membrane is a tri-layer comprising a phospholipid monolayer; and a phospholipid bilayer.

Milk fat globules, preferably of bovine source, to be used for the composition according to the disclosure can be obtained by using as source fresh (raw) non-skimmed bovine milk, containing still its full fat content (i.e. cream). By routine dairy treatment procedures the fat fraction of the milk, containing the milk fat globules, can be isolated. Thereby, basically, the difference in density between the milk fat and (non-fat) skim milk fractions effectuates passive separation of the lighter milk fat fraction containing the milk fat globules, which thus rise to the top when whole milk (containing all fat) is left undisturbed for a time interval of e.g. 24 h. By common diary techniques, the cream separation can be actively accelerated applying centrifugal force using a centrifuge, also called (milk) separator. After isolation, if desired, cream fractions can be washed with water or salt solution to (further) remove casein and whey proteins.

Instead of raw milk as basic source for the milk fat globules it is also possible to use homogenized milk, which contains milk fat globules that on average are reduced in size, accomplished by the homogenization process which is applied on (raw) milk to avoid creaming (i.e. rising of fat globules to the top based on the aforementioned density difference). Thus, milk fat globules obtained from homogenized milk will have a profoundly reduced average diameter: from on average between 2 and 12 µm the globule size is reduced to < 2 µm on average. Precise fat content, and consequently the milk fat globule fraction, in harvested cream fractions can be analyzed with routine dairy techniques such as the Gerber fat determination method.

Dairy techniques are constantly evolving and sophisticated separation of milk fat globule membranes is currently possible [93]. Therefore, it is feasible to prepare the compositions according to the invention using as basic components a fraction of isolated milk fat globule membranes in combination with triglycerides, being the source for the fat core of the lipid vesicle carrier, and the pentacyclic triterpenoid of the invention. A clear envisioned disadvantage of such an artificially composed triterpenoid-containing lipid vesicle with a tri-layered membrane is obviously its more laborious preparation and the related (much) higher costs, when compared to natural isolated milk fat globules as basic source of the vesicles according to the invention.

Therefore, the preferred composition comprising a pentacyclic triterpenoid compound according to the disclosure, contains one pentacyclic triterpenoid compound, or a combination of two or more different pentacyclic triterpenoid compounds, being comprised in a plurality of (natural) milk fat globules.

The lipid vesicle(s) or globule(s) comprised in the composition of the disclosure, preferably milk fat globules, may have a (median or average) diameter of between 0.1 and 15 µm, preferably between 1 and 10 µm, more preferably between 2 and 6 µm as can be determined by laser diffraction. In the present disclosure, median diameter is preferably determined by laser diffraction measurement, e.g. of the respective lipid vesicle(s) or globule(s) comprised in the composition (or sample thereof), wherein the D50 percentile value of such measurement represents the said median diameter. The D50 value can be described as the value of the particle diameter at 50% in the cumulative volume-weighted particle size distribution as determined by laser diffraction. Alternatively, average diameter (i.e. mean diameter) can be determined by laser diffraction measurement, e.g. of the respective lipid vesicle(s) or globule(s) comprised in the composition (or sample thereof), preferably by calculating the volume-weighted average (i.e. mean) particle size diameter (mostly symbolized as D[4,3], and also called De Brouckere mean diameter) with the formula D[4,3] = (Σnᵢ.dᵢ⁴)/(Σnᵢ.dᵢ³). In other words, the mean diameter herein preferably refers to a summation of all particle diameters measured to the fourth power (1 - n particles, dᵢ⁴ indicates (diameter of the i^{th} particle)⁴) in the numerator, divided by a summation of all particle diameters measured to the third power (1 - n particles, dᵢ3 indicates (diameter of the i^{th} particle)³) in the denominator. Laser diffraction data are by nature volume-weighted as the diffraction of light is proportional to the volume of the particle and thus laser diffraction delivers volume-weighted results. The skilled person is well-acquainted with laser diffraction measurements for determining average or mean particle diameter. For example, this may be performed with a Bettersizer S3 Plus instrument (Bettersize Instruments Ltd, Dandong, China) or with a Mastersizer^{tm} 3000 instrument (Malvern Panalytical), according to manufacturer's instructions. In such measurement, a laser beam is directed through a sample, and the scattered light is analyzed to determine the diameter distribution of particles. The diffraction pattern provides information about the particle diameters present, allowing calculation of the average or mean diameter.

A slightly reduced milk fat globule size and more narrow size distribution may have the advantage that the stability of the formulation is better preserved. On the other hand, a bigger average size of the milk fat globules (or milk fat globule-based vesicles) may advantageously add to the final triterpenoid payload in the formulation. Therefore, a trade-off effect likely exist between on the one hand big milk fat globules (or their derived vesicles) and smaller milk fat globules on the other hand. Depending on the precise application and its related end-product the preferred choice can be made for either the big milk fat globules (with a wide size distribution) or those on average reduced in size, such as those obtained with afore described homogenization procedures. It is anticipated that the somewhat smaller sized milk fat globules, with more narrow size distribution, possess a overall preferred combination of characteristics.

The pentacyclic triterpenoid compound may be a triterpenoid chosen from the group of lupane-type pentacyclic triterpenoids, from the group of oleanane-type pentacyclic triterpenoids or from the group of ursane-type pentacyclic triterpenoids.

Preferably the pentacyclic triterpenoid compound according to the disclosure is betulinic acid, and/or betulin, and/or lupeol, and/or oleanolic acid and/or ursolic acid. More preferably the pentacyclic triterpenoid compound is betulinic acid and/or betulin, and most preferably the compound is betulinic acid.

Support for the composition according to the disclosure presented herein is mostly effectuated in experiments with betulinic acid. However, as the other preferred pentacyclic triterpenoid compounds of interest own a very related chemical structure, the milk fat globule drug composition is highly likely also applicable as favorable drug carrier system for these compounds. Experimental support for the latter compounds (betulin, lupeol, oleanolic acid and ursolic acid) will therefore be highly likely obtainable in further elaborations of the experiments.

The composition of the present invention containing the triterpenoid compound and, as carrier system, the lipid vesicle or globule, preferably milk fat globule, is preferably comprised in a liquid emulsion formulation comprising a multitude of said lipid vesicles, globules or milk fat globules, for example at least 10⁴, 10⁵, 10⁶, 10⁷, 10⁸, 10⁹, 10¹⁰, 10¹¹, 10¹², 10¹³ per milliliter formulation.

The liquid emulsion formulation according to the disclosure preferably comprises at least 10, 15, 20, 25, 30, 35, 40, 50, 60, 70 mg/ml (pentacyclic) triterpenoid compound of which preferably at least 50, 60, 70, 80, 90 wt.% is surrounded by a trilayer membrane and/or incorporated in the milk fat globules or lipid vesicles (relative to the total weight of triterpenoid compound in the formulation).

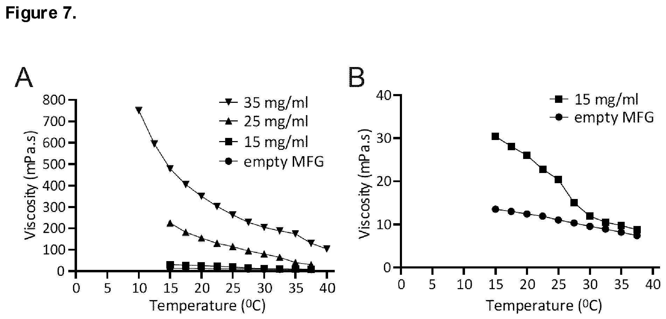

It is obvious that, by using milk fat globules obtained from the cream fraction of milk as source excipients for the preparation, the initial composition according to the disclosure will have a liquid aspect. The viscosity of this liquid formulation is increased by the incorporation of the triterpenoid compound, as elaborated in example 4 and shown in Fig. 7. This is considered an advantageous effect as it will improve stability of the formulation. In part, viscosity may as well be influenced by the preparation method, as sonication influences milk fat globule size and possibly also membrane composition.

Alternatively and being another preferred embodiment, the composition according to the current disclosure comprising the (pentacyclic) triterpenoid compound incorporated in the lipid vesicle, preferably a milk fat globule, may be in the form of a powder formulation. The composition comprising the pentacyclic triterpenoid compound in a powder form is preferably obtained by spray-drying or freeze drying procedures applied upon the liquid formulation as described above.

Spray-drying involves the transformation of a fluid material into dried particles, taking advantage of a gaseous hot drying medium. This method is used on routine base for preparation of dairy products.

Freeze-drying (or lyophilization) is an alternative method of drying in which water is removed from a fluid material by freezing it and removing the ice by placing the product under a vacuum, allowing the ice to change directly from solid to vapor. Both methods of drying have relative to each other advantages and disadvantages, related to, among others, the quality of the product, including possible structural changes which may occur, the complexity of the procedure and costs of the equipment.

After drying, subsequent powder forms of the triterpenoid composition of the invention can be used for oral consumption (i.e. oral administration) after their solubilization (rehydration) in beverages or in medicinal liquid or syrup products. Alternatively, the powder form embodiment of the composition of the invention can be used in the preparation of edible (food) products and/or dietary supplements, for instance in the form of energy, protein and granola bars or baked goods such as cookies, cakes, pastries, sauces and dips. It is also conceivable to orally administer the powder embodiment of the invention after their composition into a tablet- or capsule-type formulation, although from the perspective of uptake in the gastrointestinal system this option is less favorable.

In general, the triterpenoid composition according to the disclosure in a powder form may have as advantage that the shelf-life is extended, storage conditions may be less restricted and/or transport conditions are less cumbersome than those of the liquid triterpenoid formulation.

In a preferred embodiment, the composition comprising the triterpenoid compound according to the present invention, e.g. as comprised in the tri-layered lipid vesicle or milk fat globule, is for oral administration and/or comprised in a food composition, beverage composition, food supplement composition, dietary composition, nutraceutical composition or pharmaceutical composition.

As such the formulation according to the invention may be used in the form of a beverage for direct oral administration and consumption, it may be mixed into another beverage before oral administration, or it may be used in the preparation of baked or otherwise-prepared food products serving as dietary supplement or nutraceutical.

Alternatively, in another preferred aspect, the formulation containing the triterpenoid compound as comprised in the tri-layered lipid vesicle or milk fat globule according to the disclosure, can be applied in the form of an ointment or cream suitable for topical administration on the skin, e.g. to treat melanoma or skin disorders or for cosmetic purposes. The administration on the eyelids of one aspect of the triterpenoid composition according to the disclosure in the form of an ophthalmic ointment or cream may be used for ocular application.

As the excipients of the invention are natural and food-derived in origin and known for their optimal gastrointestinal uptake and absorption, it is highly likely that upon oral administration the triterpenoid formulation of the invention will be able in providing favorable bioavailability of said triterpenoid compounds.

By the nature of its excipients, the topical application of said triterpenoid-milk fat globule formulation of the invention is expected to also provide sufficient dermal bioavailability of the triterpenoid compounds, enabling favorable bioactive effects for skin-related diseases and/or improving dermatological conditions..

As elaborated in the preceding sections of this application, the pentacyclic triterpenoids of interest, in particular betulinic acid, betulin, lupeol, oleanolic acid, and/or ursolic acid, have known biological effects rendering the compounds suitable for therapeutic use, either alone or in combination regimes with other chemotherapeutical compounds, in particular for the treatment of cancer. As such, the pentacyclic triterpenoids, preferably betulinic acid, can be used therapeutically to reduce tumor growth, ameliorate cancer-related health deterioration, and/or can be used as a priming active pharmaceutical ingredient to enforce the effects of other chemotherapeutics for caner, e.g. by counter-forcing (limiting) drug-resistance. Many other beneficial effects are known for the triterpenoid compounds described herein, as elaborated before. Therefore, other diseased health states likely will benefit as well from treatment with any of the herein described pentacyclic triterpenoids being comprised in the composition of the disclosure, in particular betulinic acid. The known effects of in particular betulinic acid selectively on cancerous cells, reducing their proliferation, render the pentacyclic triterpenoid compositions of the invention also exquisitely suitable for the use as (chemo)preventive measure to avoid development of cancer. Such an use can be envisioned in the form of a dietary (food) supplement or nutraceutical. The use as dietary supplement of the composition as described herein is in particular also expected to be useful to improve health in its general (not necessarily diseased) aspects; e.g. the composition of this invention may be useful for therapeutic use, e.g. to counter-act obesity, as supported by the literature, but is also anticipated, as also deduced from preclinical in vitro and in vivo art, to be useful for the prevention, amelioration or treatment of one or more of the following health-affecting conditions and states: diabetes type 2, cardiovascular disease and/or its preceding phases, skin disorders, chronic inflammation, and motor impairments associated with Parkinson disease. The proven effects, both in vitro and in preclinical animal experiments, of preferably betulinic acid, but also the other pentacyclic triterpenoids as described herein, against a multitude of cancer types, including those with the highest prevalence in man and women (lung cancer, colorectal cancer, breast cancer, prostate cancer, cervical cancer or melanoma) render the application as pharmaceutical or food-supplement (or nutraceutical) of the composition as described herein against cancer or to prevent cancer the most preferred.

Accordingly, there is provided for the composition comprising a pentacyclic triterpenoid compound according to the invention for therapeutic use, e.g. for use in the treatment or prevention of cancer, type 2 diabetes mellitus, obesity, metabolic syndrome, cardiovascular disease, skin disorder, (chronic) inflammation, or Parkinson's disease and/or related motor impairment, preferably for use in the treatment or prevention of cancer. The cancer may be lung cancer, colorectal cancer, breast cancer, prostate cancer, cervical cancer or melanoma.

In the comprehensive study leading to the current invention, it was surprisingly found according to the present invention that by the use of high energy emulsification preparation techniques, in particular sonication in combination with high shear homogenization, a drug carrier system consisting of milk fat globules, being natural lipid vesicles with a tri-layer membrane and a triglyceride core, enable an unexpectedly high payload of triterpenoid compound to be taken up, incorporated and encapsulated, in particular in comparison to other known (lipid based) drug carrier formulations of the triterpenoids of the invention (as elaborated in table 1).

Next to the composition itself, the present invention further provides for a method of preparation of the (pentacyclic) triterpenoid compound composition, i.e. as comprised in a (milk) fat globule or lipid vesicles according to the present invention, wherein the method comprises the steps of:
a) providing a composition comprising lipid vesicles having a tri-layer membrane comprising a monolayer of phospholipids together with a bilayer of phospholipids, preferably milk fat globules itself;
b) providing the (pentacyclic) triterpenoid composition of the invention comprising one or more (pentacyclic) triterpenoid compounds incorporated into the lipid vesicles, preferably milk fat globules, by mixing the composition provided in a) with one or more different (pentacyclic) triterpenoid compounds, preferably by applying high shear mixing and/or sonication and/or high pressure homogenization technique, thereby achieving the incorporation of the triterpenoid compound into the lipid vesicles, preferably milk fat globules, and the assembly of milk fat globules, or lipid vesicles, incorporating the triterpenoid compound.

In a preferred embodiment, in step b) the method of preparation thus comprises one or a combination of two or three high-energy emulsification and homogenization methods like sonication, high shear mixing and high pressure homogenization, most preferably sonication. These techniques separately or in a combination, by mixing and agitating the particles and/or triterpenoid compounds, greatly promote the efficient and rapid incorporation at high payload of the triterpenoids into the lipidic vesicles of the invention, preferably milk fat globules, and these techniques can as such being considered ideal for the complete merging of the triterpenoid compound into the lipid vesicle, preferably the milk fat globule.

A high shear mixer, as applied herein, is a high-performance dispersing instrument applying shear forces based on the rotor-stator principle. The rotor's high circumferential spinning speed, ranging from e.g. 3000 rpm up to 35000 rpm provides in combination with the stator for the necessary shear forces, capable to break down and mix the components to be mixed, thereby e.g. emulsifying liquids and/or dispersing powders and other materials into liquids. Accordingly, a high shear mixing as used herein can be mixing by means of a device that uses a rotor to mix and the device typically consists of a rotor and stator, with the rotor capable of spinning to create shear forces. As said, rotor speeds ranging from e.g. 3000 rpm up to 35000 rpm may be used.

The high shear mixing is especially useful and may therefore especially be used as a first dispersion step in combination with a subsequent preparation step involving sonication and/or high pressure homogenization, preferably sonication.

In the process of (ultra)sonication, as preferably applied in step b, an ultrasound transducer may be used to provide ultrasonic vibration. Sonication is a process that uses sound waves to disrupt and mix components. The process is typically carried out using a sonicator, which is an instrument that generates sound waves and directs them into the components to be mixed. Ultrasound transducers exist of different types, like the horn (also called, probe) transducers, piezoelectric plates and langevin-type transducers [94]. In the present invention, preferably by using a horn (probe) type sonicator, cycles of ultrasound induced pressure cause the formation of microscopic vacuum bubbles in the solution. The bubbles continuously collapse in a process known as cavitation, which causes powerful waves of vibration with high energy applying various physicochemical effects upon particles in emulsion and causing enhanced and accelerated incorporation of the triterpenoids into the milk fat globules or milk fat globule-based vesicles of the compositions of the invention.

In addition or alternatively, step b) may involve the use of a high pressure homogenization technique which means applying at least 800 bar up to 4000 bar to force the (mixed) composition(s) through a nozzle type of aperture, having a diameter of between 10-10000 nm, or using a related high pressure homogenization instrument.

In addition or alternatively, step b) may involve heating the (mixed) composition(s) to a temperature of between 30 and 90 degrees Celsius, and/or step b) may be performed at such temperature.

The at least one triterpenoid compound of the present invention may advantageously be incorporated in milk fat globules which are in combination with (natural milk-derived) casein micelles and/or whey proteins, which may be adhered to the milk fat globules and may thereby provide a more homogenous and stable emulsion.

Such an addition of natural milk proteins to milk fat globules, or the lipid vesicles based upon milk fat globules, may be executed preferably before the preparation of the composition of the invention, but can alternatively also be accomplished after the preparation. Depending on the treatment of the source of milk fat globules used, as discussed before herein, the milk proteins may still be present in the milk fat globules emulsion used for the preparation of the composition. As elaborated in a previous section of the present application, both casein and whey proteins, of which a multitude of variants exist in milk, are known in the literature for their capacity to improve emulsification processes and may as such advantageously help to prepare and stabilize the formulation.

Alternatively, also with the aim to further improve the stabilization of the composition of the invention, non-milk derived stabilizers, preferably carrageenan, which is a mixture of natural sulfated polysaccharide extracted from red edible seaweeds and is approved for food application (with the European food additive number E407) may also be beneficially added, either before or after preparation of the composition, and either together with the milk proteins or separately.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

**The following examples illustrate the different embodiments of the invention.**

### Legends of Figures

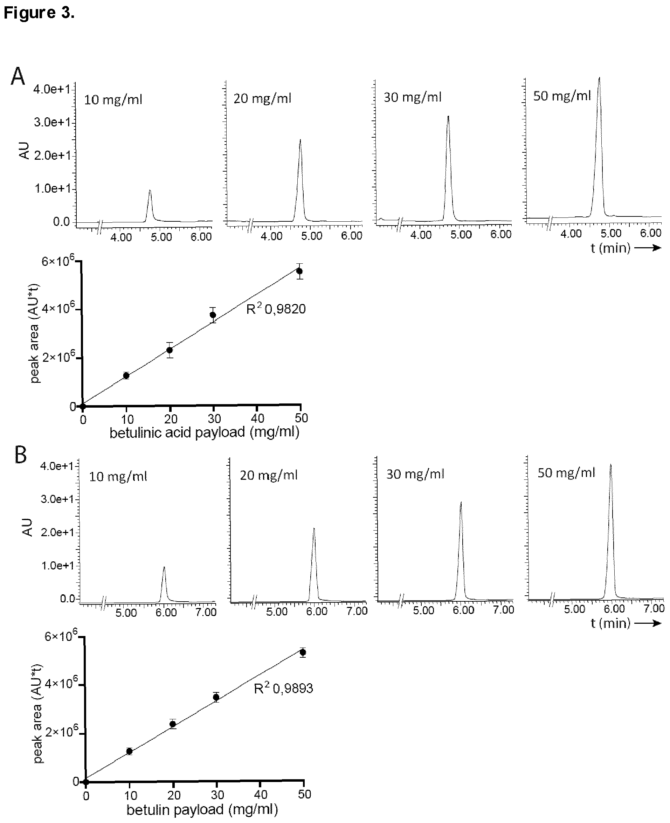

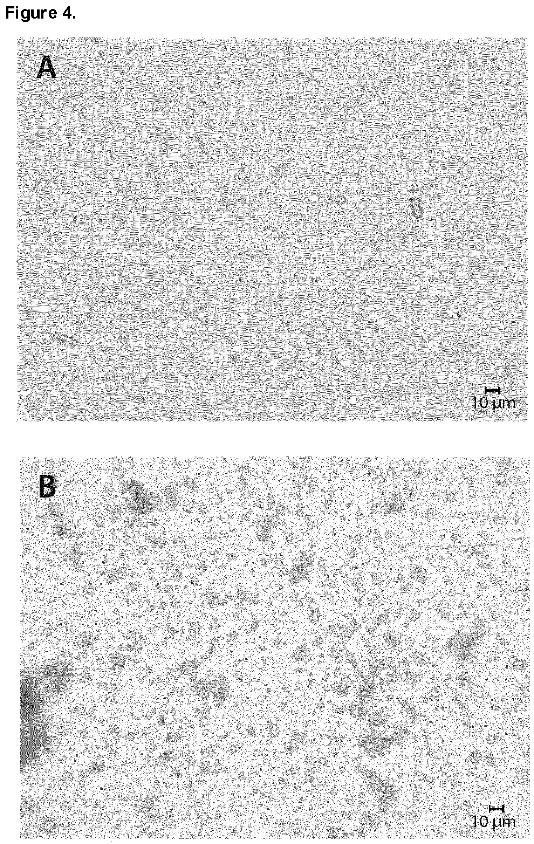

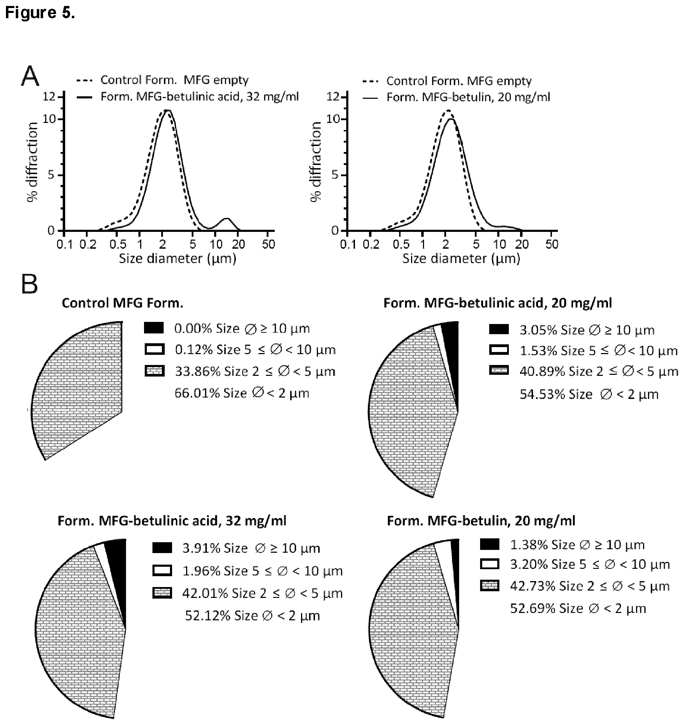

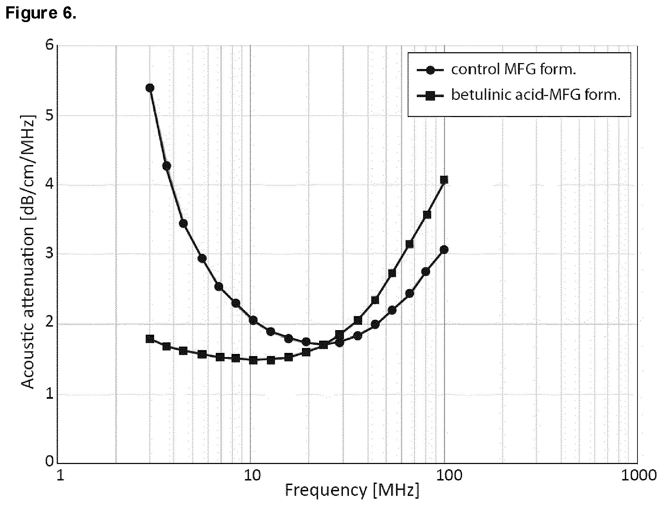

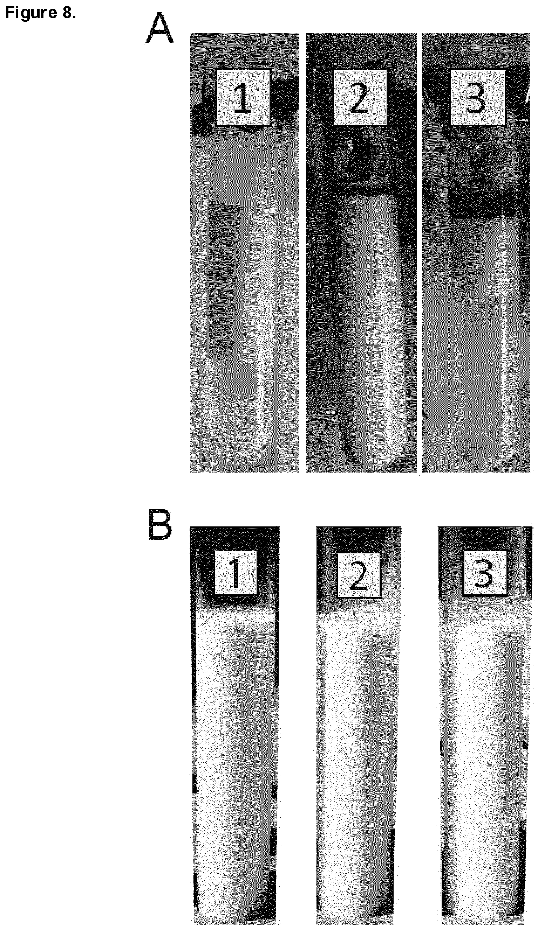

**Legend** **Figure 1****:**
   **Chemical structures of five pentacyclic triterpenoids addressed in the invention.**
   The five pentacyclic triterpenoids with profound pharmacological effects of which the drug formulation is addressed in the current patent application.
   Betulinic acid is from the lupane sub-class, its IUPAC name is (3β)-3-Hydroxylup-20(29)-en-28-oic acid, with chemical formula C30H48O3. Betulin is from the lupane sub-class, its
   IUPAC name is (3β)-Lup-20(29)-ene-3,28-diol, with chemical formula C30H50O2. Lupeol also has a lupane type scaffold, its IUPAC name is (3β)-Lup-20(29)-en-3-ol, with chemical formula C30H50O. Oleanolic acid has an oleanane scaffold, its IUPAC name is (3β)-3-Hydroxyolean-12-en-28-oic acid, with chemical formula C30H48O3. Ursolic acid is from the ursane sub-class, its IUPAC name is (3β)-3-Hydroxyurs-12-en-28-oic acid, with chemical formula C30H48O3.
**Legend** **Figure 2****:**
   **Principle of efficient assembly of milk fat globule (MFG) containing pentacyclic triterpenoid and triterpenoid incorporation at high payload.**
   In Figure 2 thickness membrane not to scale, lower half MFG: without triterpenoid. Hydrophobic triterpenoid drug incorporation highly likely occurs both in the tri-layered membrane of the milk fat globule (MFG) and in the triglyceride MFG core. As compared to smaller carrier systems, like big liposomes or nanosized liposomes, which are depicted as examples relatively to scale with MFG, there is ample more space for incorporation of bioactive triterpenoids in the MFG carrier. Both the membrane surface area of a 200 nm diameter nanoparticle is a factor 400 smaller than of a MFG vesicle with 4 µm diameter size and the core content of such a MFG is a factor 8000 more voluminous than the core of a 200 nm ø nanoparticle. Moreover, the aqueous core of a liposome vesicle is not available for loading of hydrophobic compounds.
   Depending on the relative contribution of the membrane and core to triterpenoid loading in the MFG, which is not precisely known, this renders the total drug loading capacity of one nanoliposome vesicle (ø 200 nm) a factor 500 to 1600 lower than that of one MFG vesicle (ø 4 µm), at a distribution of 20% to 75% of the triterpenoid compound loaded in the core of the MFG, respectively. It's extremely high drug loading capacity is the primary advantage of the MFG as (oral) drug delivery system. When contained in emulsions even a much higher density of nanoparticles (per volume unit) is not expected to significantly compensate for the much lower drug loading capacity per nanoparticle.
**Legend** **Figure 3****:**
   **RP-HPLC analysis of pentacyclic triterpenoid milk fat globule (MFG) formulations according to the invention.**
   Panel A: MFG-betulinic acid formulations. Panel B: MFG-betulin formulations.
   Upper panels (both in A and B): Representative RP-HPLC elution profiles of samples taken from betulinic acid or betulin - milk fat globule formulations with ascending triterpenoid payloads of 10, 20, 30 and 50 mg/ml, showing highly efficient incorporation of the triterpenoid compound over this concentration range.
   Lower panels (both in A and B): Peak area analysis with linear regression of chromatography elution profiles of four samples (n=4; standard deviation, SD, error bars indicated at each payload concentration) of the formulations with ascending triterpenoid concentration, as indicated. A strong linearity of drug incorporation is observed in this range of payloads. The four samples (n=4) were taken without prior mixing from different positions in the formulation to test within the formulations possible unequal triterpenoid incorporation (or distribution) during preparation and/or instability phenomena evolving during storage, e.g. coalescence and/or phase separation (demulsification).
**Legend** **Figure 4****:**
   **Microstructure of the betulinic acid - milk fat globule formulation according to the disclosure in comparison to a control formulation by optical microscopy.**
   Images of bright field microscopy at 100x magnification of (A) the control formulation of betulinic acid dispersed into bovine skimmed milk, which lacks milk fat globules and (B) the composition according to the invention comprising betulinic acid, at a payload of 32 mg/ml, incorporated into milk fat globules. The dispersion and emulsification procedures were completely identical for the preparation of (A) and (B). In [A], clearly rod-shaped non dissolved deposits of betulinic acid are observed, which are completely absent in the composition of betulinic acid comprised in milk fat globules.
**Legend** **Figure 5****:**
   **Particle size distribution analysis of the triterpenoid milk fat globule composition of the invention measured with laser diffraction.**
   Laser diffraction experiments were performed using the Bettersizer S3 Plus particle size analyzer (Bettersize Instruments Ltd, Dandong, China). Formulations without triterpenoids (control) or containing triterpenoids (betulinic acid or betulin) were compared.
   Panel A: Left, the betulinic acid - milk fat globule composition (betulinic acid payload, 32 mg/ml) was compared to the control milk fat globule formulation without betulinic acid but which has undergone the same preparation procedure (high shear mixing, with sonication); Right, the composition of betulin incorporated into milk fat globules (betulin payload, 20 mg/ml) was compared to the control milk fat globule formulation lacking betulin, but which has undergone the same preparation procedure (high shear mixing, with sonication).
   Panel B: the same experiments as in panel [A] expressed in pie chart diagrams with the aim to better visualize differences in particle size distributions: the % particles per size category are indicated.
**Legend** **Figure 6****:**
   **Triterpenoid-milk fat globule formulation characteristics as analyzed by acoustic spectroscopy.**
   Acoustic spectrometry experiments of a control milk fat globule formulation, lacking betulinic acid, and the composition of the invention comprising betulinic acid incorporated into milk fat globules (MFG) are shown, as indicated. Where the control milk fat globule formulation does not contain betulinic acid, it has undergone the same preparation procedure as the MFG-betulinic acid formulation, being high shear mixing and sonication. Measurements were performed at controlled temperature. A profound difference in the attenuation spectrum when betulinic acid is incorporated in the MFG carrier was observed.
**Legend** **Figure 7****:**
   **Viscosity profile at defined temperatures of betulinic acid - milk fat globule formulations.**
   The viscosity of three betulinic acid - milk fat globule compositions with increasing payloads of betulinic acid was tested together with a control formulation of unloaded milk fat globules (empty MFG). The viscosity was measured under strictly controlled temperature conditions, as viscosity and temperature are strongly interdependent. The betulinic acid payloads of the formulations are indicated in the graphs (15, 25 and 35 mg/ml). Panel A depicts all three betulinic acid formulations with the control. Panel B depicts only the 15 mg/ml formulation together with the 'empty' control, to better visualize the dynamic effects and enhanced viscosity due to the incorporation of betulinic acid, which also occurred at low betulinic acid concentration.
**Legend** **Figure 8****:**
   **Stability of betulinic acid - milk fat globule formulations after centrifugation and after long term storage.**
   Panel A. Effects of centrifugation on emulsion stability by measuring the centrifugation-induced disintegration of the betulinic acid - milk fat globule composition in comparison to control emulsions. The composition comprising betulinic acid, at 35 mg/ml payload, assembled in milk fat globules, according to the disclosure, was centrifuged at 3400 g for 50 min (Tube 1). Control emulsions were identically centrifuged and contain untreated O/W emulsions of milk fat globules derived from either homogenized milk fat globules with a size distribution of 0.2 - 2 µm (Tube 2), or from cream of raw non-homogenized milk containing milk fat globules that are considerably bigger, with a size distribution of 1 - 10 µm (Tube 3). Panel B. Shelf life stability of milk fat globule - betulinic acid formulations according to the invention after storage of 3 months at 4 °C. The composition according to the invention of betulinic acid incorporated in milk fat globules (MFG) is shown in different variants. Tube 1: MFG derived from homogenized milk, with a betulinic acid payload of 25 mg/ml; Tube 2: MFG derived from homogenized milk, with a betulinic acid payload of 40 mg/ml. Tube 3: MFG derived from raw milk, with a betulinic acid payload of 25 mg/ml.

### Experimental section

### EXAMPLE 1

### Preparation of the triterpenoid milk fat globule composition according to the invention Source of milk fat globules

The compositions according to the invention were prepared using a basic protocol. Several variants thereof, with respect to the precise source and content of the used excipients and the details of the preparation protocol, were executed. Basically, milk fat globules to be used as source for the compositions were either derived from homogenized milk cream, as supplied commercially by various dairy product companies, or milk fat globules were taken from raw (fresh) milk, as supplied by e.g. (biological) dairy farms. The raw milk cream was collected (or 'skimmed') according to basic dairy procedures, as outlined in brief before, to obtain the milk fat globules as contained in the cream fraction.

Both these sources of milk fat globules could then be used in the preparations of the compositions according to the invention, thereby taking into account shelf life of the source. Compositions prepared using milk fat globule from these two sources, each owning different average globule sizes as outlined before, were similar with respect to the payload of pentacyclic triterpenoid incorporation into the milk fat globule drug carrier system.

### Formulation preparation, method and materials

Dairy cream preparations comprising milk fat globules at high density (at least 30% milk lipids, v/v) in volumes between 20 and 100 ml were used as excipient source for the compositions of the invention. Isolated pentacyclic triterpenoid compounds at high purity (> 98%, as confirmed by mass spectrometry), were added in powder form at a range of concentrations (expressed in mg triterpenoid per ml excipient, e.g. 10, 20, 30, 40 or 50 mg/ml). For control compositions, triterpenoid compound was not added, but the preparation protocol followed was identical as been used for the triterpenoid compositions (in a pair of compositions to be compared).

For most preparations, a first dispersion step involving high shear mixing at 15000 - 20000 RPM for 2 to 5 min (equipment: Ultra Turrax dispenser, model T25, from IKA) was applied, to accomplish a first rough dispersion of the triterpenoid powder throughout the entire content of the excipient volume used. However, for some other preparations the first high-shear mixing step was left out, as it appeared to be dispensable if the second sonication phase was executed according to the most preferred steps and parameter settings.

During the second preparation phase, a sonication procedure was followed that appeared to be indispensable to ensure proper and complete entrapment and incorporation of the pentacyclic triterpenoid in the milk fat globules. Thus, sonication was used in the preparation of all formulations according to the invention, otherwise it appeared impossible to accomplish a full triterpenoid incorporation at high payloads. The sonication was accomplished with an ultrasonic probe type of processor (also coined tip, horn or pole type sonicator). The equipment used was the ultrasonic processor model FS150-N for small volume preparations and sonicator model FS1800-N for larger volumes (Zhengzhou TCH Instrument Co, Zhengzhou, China), with adjustable, respectively 80 Watt and 1800 Watt maximal output power and a frequency of 20 KHz. The following sequence of sonication cycles and parameter setup was used for the 80 Watt sonicator (FS150-N), using a probe of ø 8 mm. Formulation volumes between 25 ml and 40 ml were used and 5 cycles of sonication were applied with increasing power usage, starting at 35% power intensity, subsequently cycles at 70%, 80%, 85% and 90% power intensity. Each cycle had a duration of 5 min and within every cycle the sonication proceeded with 10 sec sonication pulses alternated with 5 sec rest time between the pulses (in total 20 pulses of 10 sec per cycle of 5 min). The primary reason to use this setting was to not excessively rise the temperature over 60 °C in the composition under preparation. Although the rise in temperature may help the emulsification process and the entrapment of the triterpenoid compound into the milk fat globule, a temperature which is too high may disadvantageously lead to denaturation of proteins in the milk fat globule membrane. Therefore, it was chosen to stay within safe temperature limits. The temperature was monitored between the cycles and during sonication within cycles. Thus, the applied cycle and pulse settings accomplished that the temperature of the formulation never raised higher than 60 °C. The sonication probe tip immersion depth applied was ≈25% under the surface of the total formulation height. After the sonication procedure formulations were adapted to room temperature and subsequently stored at 6 - 8 °C until usage.

It is anticipated, as will be appreciated by a person skilled in the art, that instead of sonication the use of high pressure emulsification techniques may similarly accomplish complete incorporation of the pentacyclic triterpenoid compounds in the milk fat globules. Likewise, in some embodiments of the composition of the invention it can be envisioned that the combination of sonication together with high pressure emulsification techniques during the preparation may advantageously help in the scaling up process needed for high volume formulation production [94].

It is worthy of note with respect to the preparation method applied in the current invention that in any publication wherein milk fat globules were used for the incorporation of active biological compounds the method of entrapment did not comprise in any sense the use of sonication or another high energy emulsification technique, such as high pressure emulsification or high shear mixing. Instead, solely simple co-incubation was applied (i.e. stirring at temperature of 25 °C) [83] or, alternatively, organic solvents were used to aid in the incorporation process [81,82].

As shown in Example 2, and proceeding likely according to the assembly principle illustrated in Fig. 2, the here described method of preparation successfully incorporated triterpenoids, preferably betulinic acid and betulin, at high payload in milk fat globules. Emulsification and incorporation of triterpenoid compounds into milk fat globules using several tested low-energy emulsification methods (e.g. ON stirring at 40 °C) were not successful, also indicating the importance of the applied high-energy emulsification techniques described herein.

### EXAMPLE 2

### Efficient incorporation of triterpenoid compound in milk fat globules as revealed by RP-HPLC and microscopy

### Sample preparation method of triterpenoid - milk fat globule composition

A two phase liquid-liquid extraction (LLE) method, using methanol (MeOH) and iso-octane as immiscible organic solvents, was developed to separate the pentacyclic triterpenoid compound from its sample matrix, being the milk fat globule constituents. In brief, samples of the triterpenoid - milk fat globule formulations were taken with a sampling spatula, at least at four different positions in one formulation (contained in a 50 ml storage vial), and samples were weighed. The two phase liquid extraction system was composed, containing MeOH to dissolve the triterpenoid (betulinic acid or betulin) and iso-octane to dissolve the matrix lipids from the milk fat globules. To that end, equal volumes (7 ml) of MeOH and iso-octane were taken and 3% (volume) water was added to advantageously modify the liquid-liquid equilibrium between the two organic solvents. The sample taken from the formulation was added to the two-phase LLE system. Formulation samples were chosen to be approximately 200 mg. After a series of samples of which the triterpenoid content had to be quantitatively compared on HPLC was taken, with mostly slightly varying weights, the volumes of MeOH and iso-octane were, if needed, adapted to obtain equal mg of formulation per ml of two-phase LLE organic solvent system. The sample was strongly mixed in the LLE system using the vortex technique. Subsequently, the LLE system was heated to 40 °C and left for 30 min to help sample disintegration. Then to further improve the dissolution of the sample, the LLE system with sample was vigorously mixed with a high shear mixer at 10000 RPM (with an Ultra turrax, model T25, high shear mixer, which works according to the rotor stator principle), and thereafter left undisturbed for 2 hours at 40 °C to reinstall the phase separation between the MeOH and iso-octane solvent layers.

From the underlying MeOH phase, containing the dissolved triterpenoid, a sample of 1.2 ml was taken and centrifuged at 10,500 RPM for 5 min to remove any possible debris, subsequently 300 µl samples were taken for HPLC analysis. Quantitative RP-HPLC analysis of thus-prepared formulation samples for HPLC, as compared to a concentration series of triterpenoid (betulinic acid or betuline) standard samples, in conjunction with standard addition experiments, revealed that the here described sample preparation procedure leads on average to a 30% loss of triterpenoid content (data not shown).

### RP-HPLC analysis of triterpenoid compounds, method

RP-HPLC analyses were performed using a chromatographic system consisting of a Waters Alliance 2695 Separation Module coupled to a Waters 996 Photo Diode Array detector. The stationary phase was a C-18 column (Waters Symmetry^{®} C18, 5µm 4.6 x 250 mm). The injection volume was 20 µl and the flow rate was 1.5 ml/min under controlled temperature of 30 °C. For separation, a mobile phase composed of acetonitrile-water at a ratio of 91:9 (v/v) was used for elution in isocratic mode. The detection wavelength of the PDA detector was set at 210 nm. The chromatographic peaks of betulinic acid and/or betulin were confirmed by comparing their retention times with betulinic acid and betulin standards of > 98% purity (Sigma-Aldrich), dissolved in methanol.

### RP-HPLC analysis of triterpenoid - milk fat globule formulations, results

The HPLC chromatography of samples prepared from the compositions of betulinic acid in milk fat globules and betulin in milk fat globules efficiently detected the triterpenoid compounds in the formulations (Fig. 3.A and B, upper panels, representative elution profiles are shown. Note the difference in retention time of betulinic acid and betulin), and showed linearity of triterpenoid incorporation, also at high payloads (Fig. 3.A and B, lower panels). The complete incorporation of betulinic acid and betulin into the milk fat globule was deduced from different findings. The emulsion formulations of betulinic acid or betulin in milk fat globules both were extremely stable (Fig. 8.A and B) and the betulinic acid (or betulin) was homogenously emulsified, as was indicated by the only small standard deviation between the samples prepared from 4 different positions in the emulsion, as shown in Fig. 3.A and B, lower panels. Of note: random sampling at different spots in the emulsion was executed after at least a week undisturbed shelf rest, and without prior mixing (dispersion) of the emulsions. In addition, when samples were taken after forceful centrifugation from the upper lipid fraction of the emulsion (see centrifugation as described in Example 5 and shown in Fig. 8.A, tube 1), the results were likewise, with almost identical triterpenoid concentrations observed in samples taken at different spots throughout the full lipid fraction of the emulsion, also indicating a stable and homogenous distribution of the triterpenoid throughout the formulation (data not shown). As expected, however, a higher concentration of the triterpenoid was detected in the HPLC samples from the upper lipid fraction as compared to the noncentrifuged emulsion, because the aqueous lower fraction (Fig. 8.A, tube 1), separated (and analyzed) after centrifugation, does not contain any triterpenoid (data not shown).

Moreover, microscopy of triterpenoid - milk fat globule (MFG) compositions and their comparison with control formulations of triterpenoids without MFG provided a strong additional indication that the amount of triterpenoid that initially was added during preparation of the formulation according to the invention was fully incorporated into the MFG.

### Brightfield microscopy of triterpenoid - milk fat globule compositions

The image in Fig. 4.B of brightfield optical microscopy (100x magnification, using a Kern OBL137C832 microscope, Kern & Sohn GmbH, Germany) of the betulinic acid - milk fat globule formulation (with 32 mg/ml betulinic acid payload) show a normal aspect of milk fat globules without any visible signs of either crystallized or amorphous betulinic acid deposits. On the other hand, in a control formulation of betulinic acid dispersed, according to the same emulsification procedures, in skimmed milk (Fig. 4.A), which mainly lacks milk fat globules, betulinic acid deposits are abundantly and clearly visible as rod-like structures.

The importance of this type of particle characterization by microscopy is foremost in the direct observations, without the often complicated interpretation issues of e.g. laser diffraction and acoustic spectroscopy results of complex emulsions, which are often confounding and precluding clear conclusions.

### EXAMPLE 3

### Particle size characterization of triterpenoid - milk fat globule compositions

### Particle characterization, including size distribution, measured with laser diffraction

The particle characteristics of the milk fat globule (MFG) control formulation and the MFG formulation of the invention containing bioactive pentacyclic triterpenoids were analyzed by laser diffraction using the Bettersizer S3 Plus particle size analyzer (Bettersize Instruments Ltd, Dandong, China). The control formulation does not contain triterpenoid molecules, but the preparation procedure, including high shear mixing and sonication, was performed identically as the protocol for the formulations containing the triterpenoids.

Laser diffraction measurements can be difficult to interpret with certainty under certain circumstances. The experiments shown in Fig. 5 (in panels A and B the same data are shown, but represented in a different manner, for clarity and interpretation reasons) strongly suggest that the MFG particle size is enlarged by the incorporation of the bioactive triterpenoids, because in the control formulation, MFG sized >5 µm were not apparent, whereas after incorporation of the pentacyclic triterpenoid (betulinic acid or betuline) this size category is significant. It cannot be fully excluded, however, that differences between control and triterpenoid formulations other than MFG size differences, such as in particular the occurrence of aggregates of MFG particles, is the underlying full or partial cause of the observed effect. However, all samples were pretreated identically, including a preceding short ultrasonication step to dismantle aggregates just before laser diffraction measurements, rendering the latter explanation less likely.

Also, both the volume-weighted average (i.e. mean) particle size diameter and median particle size diameter, obtained by laser diffraction, were profoundly increased in the MFG formulations containing betulinic acid when compared with the formulation containing solely the excipients, equally treated but without the triterpenoid (data not shown). This is in line with the results shown in Fig 5, indicating a particle enlarging effect of the triterpenoid. The volume-weighted average (i.e. mean) particle size diameter, with the symbol D[4,3], can be calculated using the formula (Σnᵢ.dᵢ⁴)/(Σnᵢ.dᵢ³); the median particle size diameter is synonymous with the D50 value, indication the 50% percentile particle size of the size distribution.

### Particle characterization as measured with acoustic spectroscopy

The principle of acoustic attenuation to analyze particles in concentrated emulsions involves an acoustic sensor which measures the attenuation of the ultrasound waves passing the emulsion in the frequency range from 1 to 100 MHz with variable transmitter-detector distances. To this end, the acoustic and electro-acoustic spectrometer DT-1202 (Dispersion Technology Inc., USA) was used.

In Fig. 6 a clear reciprocal effect is shown whereby the acoustic attenuation of the betulinic acid-milk fat globule pharmaceutical formulation is lower than the 'empty' control milk fat globule formulation at acoustic frequencies below 35 MHz, whereas at frequencies from 35 MHz upwards the reverse effect is observed. The profound difference in the acoustic attenuation profiles between milk fat globules with and without the pentacyclic triterpenoid is necessarily caused by the incorporation of the compound, because the preparation procedures were similar.

The detailed interpretation of these results is not without complexity [95], because next to particle size, also viscosity and particle composition may influence the attenuation spectrum and the latter two parameters can be altered by the incorporation of the bioactive triterpenoid compound as well. In general, in the low acoustic frequency range a lower acoustic attenuation correlates with larger milk fat globule particles [95], suggesting an enlargement of the milk fat globules by incorporation of the triterpenoid compound, which would also be in line with the observed results using laser diffraction (Fig. 5). However, also altered viscosity (see Fig. 7) may, at least partly, explain the observed effect.

### EXAMPLE 4

### Incorporation of betulinic acid enhances formulation viscosity

Viscosity measurements of the betulinic acid - milk fat globule formulations were performed with the sine wave vibro-viscometer SV-10 from A&D Company Ltd (Tokyo, Japan) with temperature sensor and viscosity measurement limits of 0.3 - 10000 mPa.s (milli pascal.second). This equipment is based on the sine-wave vibration method. Dynamic viscosity was measured continuously under strictly temperature controlled conditions. The formulation to be tested was put in the sample cup, which was in a water jacket with circulating water allowing a precisely controlled formulation temperature. The refrigerated bath circulator used to this end was from Thermo Scientific, type Neslab RTE-201.

The results shown in Fig. 7 indicate that the viscosity is strongly increased by the incorporation of betulinic acid, including a strong positive relation between the betulinic acid payload in the formulation and the level of viscosity enhancement. The enhanced viscosity highly likely causes an increased formulation stability, which is an additional advantageous characteristic of the herein disclosed triterpenoid composition. An enhanced viscosity is likely caused by the incorporation of betulinic acid, resembling cholesterol, in the trilayer membrane of the milk fat globules. From cholesterol it is known that it makes membranes more rigid thereby increasing viscosity [96].

### EXAMPLE 5

### Compositions according to the invention are stable

For purposes of oral or topical application, and as will be appreciated by the person skilled in the art, it is important that a to be administered formulation is stable with a homogenous composition and as well stable in time during storage. Stability was tested using two methods, centrifugation and with (simple) shelf life storage tests.

Accelerated stability testing was performed by centrifugation at high g-force. To that end betulinic acid - milk fat globule (MFG) formulations were centrifuged at 3400 g for 50 min. A profound effect of the betulinic acid incorporation into the MFG composition in conjunction with its method of preparation was observed. The betulinic acid - MFG composition showed a two phase separation after centrifugation, with a watery layer at the bottom of the tube and a homogenous lipid phase on top (Fig. 8.A, Tube 1). In contrast the untreated milk fat globule 'source compositions', obtained from either homogenized milk or raw milk (containing relatively small or big sized MFG, respectively, as described before) showed a profoundly different phase separation after centrifugation, with a three-phase separation with a clear oily layer on top (Fig. 8.A, Tubes 2 and 3. Of note, in tube 2 containing the O/W dairy emulsion of homogenized small-sized MFG, the second phase separation can only be detected under translucent light). These results suggest that the incorporation of betulinic acid into the milk fat globules, in conjunction with its preparation procedure (foremost sonication), achieves an increased (lipid particle) stability in the formulation, as the oily phase, which contains 'liberated' fats from the milk fat globule core, did not appear. This may be explained by the incorporation of betulinic acid into the MFG membrane, thereby increasing membrane rigidity and its resistance against centrifugation-induced membrane disruption.

Shelf life stability was tested after various storage intervals. Shown in Fig. 8.B are the results of a 3-month storage period at 4 °C of three betulinic acid - milk fat globule formulations with different betulinic acid payloads. In all the instances that these tests were performed the compositions as disclosed herein remained highly stable without phase separation, which if occurring would have pointed to unfavorable flocculation and/or coalescence phenomena within the emulsions. Storage temperatures of around 4 °C are commonly accepted and required conditions for dairy(-based) (food) products, and the apparent undisturbed emulsion stability after 3 months at this temperature can be considered as an advantageous characteristic of the triterpenoid - MFG formulations of the invention.

Moreover, in storage experiments at room temperature a similar stability was observed after one month, with also absence of bacterial growth in the majority of cases tested (8 out of 9 formulations tested: >85%) (data not shown). Such an apparent advantageous antibacterial effect may be explained by a joint action of the sonication procedure, with known bacteriostatic effects, and the incorporation of betulinic acid, a compound with proven antibacterial activity.

### EXAMPLE 6

### Assessment of the betulinic acid - milk fat globule composition of the invention in comparison to reported betulinic acid formulations in the art

A comprehensive assessment was performed for the most prominent reported betulinic acid formulations with pharmaceutical potential, as shown in Table 1. For this overview betulinic acid was chosen because of its chemical structure being strongly similar to the other triterpenoids addressed in the invention, causing their strong hydrophobicity, and most published formulation research efforts are directed to betulinic acid.

Drug loading capacity (payload) and an indication of the formulation excipients were assessed. The other characteristics listed, being (1) laboriousness of preparation and related cost-effective production, (2) potential toxicity and related possible regulatory constraints, (3) assessment and/or estimation of the stability of the formulation, and (4) the estimated suitability for oral administration and nutraceutical product development, were also assessed. The all over assessment of these different betulinic acid formulations as compared to the composition of the present invention, comprising a triterpenoid compound (preferably betulinic acid and/or betuline) incorporated in preferably milk fat globules, brings the composition of the invention favorable to the front by having advantageous characteristics, relative to the other formulations, on all the aspects listed in Table 1.

**TABLE 1.**

| Overview of most prominent drug delivery systems of betulinic acid reported in the literature and the composition of the invention as compared on important aspects. | | | | | | | | |
|---|---|---|---|---|---|---|---|---|
| **Formulation, in brief: carrier & excipients*^{a}*** | **Drug Loading capacity: betulinic acid contents*^{b}*** | **Reference** | **Excipients: source & synthetic excipients involved** | **Excipients: potential toxicity related regulatory constraints*^{c}*** | **Preparation effort (related to costeffectiveness)** | **Stability of formulation** | **Use: suitability for oral delivery** | **Use: versatility** for **nutraceutical product development** |
| **Milk Fat Globules-betulinic acid microemulsion** | > 40 mg/ml; 10.99 %-wt | this patent application | natural food component | no | relatively simple | ++ | ++ | ++ |

| **Liposomes containing betulinic acid:** | | | | | | | | |
|---|---|---|---|---|---|---|---|---|
| large liposomes. Size Ø 1 - 1.5 µm | ≈ 5 mg/ml | ref.9 | lipids | no | laborious | + | + | +/- |
| nano liposomes. Size: Ø 180 nm | 12.5 µg/ml | ref.97 | lipids | no | laborious | + | + | +/- |
| nano liposomes, PEG-modified. Size: Ø 142 nm | 0.5 mg/ml | ref.47 | lipids & synthetic | yes | laborious | + | +/- | +/- |

| **Nanoemulsions containing betulinic acid:** | | | | | | | | |
|---|---|---|---|---|---|---|---|---|
| nanoemulsion comprising flaxseed oil and egg phosphotidylcholine | 8.3 mg/ml | ref.98 | purified lipids | no | laborious | + | + | + |
| nanoemulsion comprising olive oil, labrasol^{⊙} and plurol isostearique^{⊙} | 0.99 %-wt | ref.99 | lipids & synthetic | yes | laborious | ++ | +/- | +/- |
| nanoemulsion of Lauroglycol FCC^{⊙}, Cremophor EL^{⊙}, Labrafil M1944CS^{⊙} | 0.63 %-wt | ref.100 | lipids & synthetic | yes | laborious | + | +/- | +/- |
| nanoemulsion of caprylic acid, Cremophor EL^{⊙}, Labrafil M1944CS^{⊙} | 0.55 %-wt | ref.100 | lipids & synthetic | yes | laborious | + | +/- | +/- |

| **Polymeric nanoparticles containine betulinic acid:** | | | | | | | | |
|---|---|---|---|---|---|---|---|---|
| PLGA-PEG co-polymeric nanoparticles | 9.89 %-wt | ref.44 | synthetic | yes | laborious | N.D. | +/- | +/- |
| PLGA-based nanoparticles (with derivative of betulinic acid) | 8 %-wt | ref.101 | synthetic | yes | laborious | +/- | + | +/- |
| galactosylated chitosan nanoparticles | ≈ 10 %-wt. | ref.102 | synthetic modified | no | laborious | N.D. | +/- | +/- |
| chitosan nanoparticles | 6 mg/ml | ref.103 | natural purified | no | laborious | N.D. | +/- | +/- |
| octadecylamine-(betulinic acid) conjugated nanoparticles | 7.89 %-wt | ref.104 | synthetic | yes | laborious | + | +/- | +/- |
| hydrogel of PEG-carboxymethylcellulose nanoparticle a-cyclodextrin | 1.98 %-wt | ref.105 | synthetic | yes | laborious | +/- | +/- | +/- |
| folate-conjugated PEG-based nanoparticles | 18 %-wt | ref.106 | synthetic | yes | laborious | N.D. | +/- | +/- |

| | | | | | | | | |
|---|---|---|---|---|---|---|---|---|
| ***(a)*** Abbreviations used: PEG, polyethylene glycol; PLGA, poly lactic-co-glycolic acid; N.D., not determined ***(b)*** Drug loading capacity (maximum) expressed in milligram (mg) triterpene (betulinic acid) per milliliter (ml) formulation and/or in percentage weight (%-wt) betulinic acid in the carrier (non-diluted). ***(c)*** Potential toxicity-related regulatory constraints if formulation is used for oral administration, as appraised from the literature and regulatory authorities | | | | | | | | |

### REFERENCES

1. Mishra BB, Tiwari VK: Natural products: an evolving role in future drug discovery. Eur J Med Chem 46:4769-4807, 2011.
2. Newman DJ, Cragg GM: Natural products as sources of new drugs over the last 25 years. J Nat Prod 70:461-477, 2007.
3. Dehelean CA, Marcovici I, Soica C, Mioc M, Coricovac D, lurciuc S, Cretu OM, Pinzaru I: Plant-Derived Anticancer Compounds as New Perspectives in Drug Discovery and Alternative Therapy. Molecules 26:1109, 2021.
4. Liu K, Zhang X, Xie L, Deng M, Chen H, Song J, Long J, Li X, Luo J: Lupeol and its derivatives as anticancer and anti-inflammatory agents: Molecular mechanisms and therapeutic efficacy. Pharmacol Res 164:105373, 2021.
5. Sun, Fang, Wang, Hu: Structure-activity relationships of oleanane- and ursanetype triterpenoids. Botanical Studies 47:339-368, 2006.
6. Margaret M.O'Connel, Michael D.Bentley, Christopher S.Campbell, Barbara J.W.Cole: Betulin and Lupeol in bark from four white-barked birches. Phytochemistry 27:2175-2176, 1998.
7. Soica CM, Dehelean CA, Peev C, Aluas M, Zupko I, Kasa P, Jr., Alexa E: Physico-chemical comparison of betulinic acid, betulin and birch bark extract and in vitro investigation of their cytotoxic effects towards skin epidermoid carcinoma (A431), breast carcinoma (MCF7) and cervix adenocarcinoma (HeLa) cell lines. Nat Prod Res 26:968-974, 2012.
8. Kessler JH, Mullauer FB, de Roo GM, Medema JP: Broad in vitro efficacy of plant-derived betulinic acid against cell lines derived from the most prevalent human cancer types. Cancer Lett 251:132-145, 2006.
9. Mullauer FB, van BL, Daalhuisen JB, Ten Brink MS, Storm G, Medema JP, Schiffelers RM, Kessler JH: Betulinic acid delivered in liposomes reduces growth of human lung and colon cancers in mice without causing systemic toxicity. Anticancer Drugs 22:223-233, 2011.
10. Lou H, Li H, Zhang S, Lu H, Chen Q: A Review on Preparation of Betulinic Acid and Its Biological Activities. Molecules 26: 2021.
11. Hordyjewska, Ostapiuk, Horecka, Kurzepa: Betulin and betulinic acid: triterpenoids derivatives with a powerful biological potential. Phytochem Rev 18:929-951, 2019.
12. Mullauer FB, Kessler JH, Medema JP: Betulinic acid, a natural compound with potent anticancer effects. Anticancer Drugs 21:215-227, 2010.
13. Pisha E, Chai H, Lee IS, Chagwedera TE, Farnsworth NR, Cordell GA, Beecher CW, Fong HH, Kinghorn AD, Brown DM, Wani MC, Wall ME, Hieken TJ, Das Gupta TK, Pezzuto JM: Discovery of betulinic acid as a selective inhibitor of human melanoma that functions by induction of apoptosis. Nat Med 1:1046-1051, 1995.
14. Pavlova NN, Zhu J, Thompson CB: The hallmarks of cancer metabolism: Still emerging. Cell Metab 34:355-377, 2022.
15. Potze L, Di Franco S, Grandela C, Pras-Raves ML, Picavet DI, van Veen HA, van Lenthe H, Mullauer FB, van der Wel NN, Luyf A, van Kampen AH, Kemp S, Everts V, Kessler JH, Vaz FM, Medema JP: Betulinic acid induces a novel cell death pathway that depends on cardiolipin modification. Oncogene 35:427-437, 2015.
16. Heiss EH, Kramer MP, Atanasov AG, Beres H, Schachner D, Dirsch VM: Glycolytic switch in response to betulinic Acid in non-cancer cells. PLoS ONE 9:e115683, 2014.
17. Epstein T, Gatenby RA, Brown JS: The Warburg effect as an adaptation of cancer cells to rapid fluctuations in energy demand. PLoS ONE 12:e0185085, 2017.
18. Reddy AS, Zhang S: Polypharmacology: drug discovery for the future. Expert Rev Clin Pharmacol 6:41-47, 2013.
19. Wang YJ, Liu JB, Dou YC: Sequential treatment with betulinic acid followed by 5-fluorouracil shows synergistic cytotoxic activity in ovarian cancer cells. Int J Clin Exp Pathol 8:252-259, 2015.
20. Pandita A, Kumar B, Manvati S, Vaishnavi S, Singh SK, Bamezai RN: Synergistic combination of gemcitabine and dietary molecule induces apoptosis in pancreatic cancer cells and down regulates PKM2 expression. PLoS ONE 9:e107154, 2014.
21. Gao Y, Jia Z, Kong X, Li Q, Chang DZ, Wei D, Le X, Huang S, Wang L, Xie K: Combining betulinic acid and mithramycin A effectively suppresses pancreatic cancer by inhibiting proliferation, invasion and angiogenesis. Cancer Res 2011.
22. Yamai H, Sawada N, Yoshida T, Seike J, Takizawa H, Kenzaki K, Miyoshi T, Kondo K, Bando Y, Ohnishi Y, Tangoku A: Triterpenes augment the inhibitory effects of anticancer drugs on growth of human esophageal carcinoma cells in vitro and suppress experimental metastasis in vivo. Int J Cancer 125:952-960, 2009.
23. Sawada N, Kataoka K, Kondo K, Arimochi H, Fujino H, Takahashi Y, Miyoshi T, Kuwahara T, Monden Y, Ohnishi Y: Betulinic acid augments the inhibitory effects of vincristine on growth and lung metastasis of B16F10 melanoma cells in mice. Br J Cancer 90:1672-1678, 2004.
24. Nakagawa-Goto K, Yamada K, Taniguchi M, Tokuda H, Lee KH: Cancer preventive agents 9. Betulinic acid derivatives as potent cancer chemopreventive agents. Bioorg Med Chem Lett 19:3378-3381, 2009.
25. Fulda S: Betulinic Acid for cancer treatment and prevention. Int J Mol Sci 9:1096-1107, 2008.
26. Cichewicz RH, Kouzi SA: Chemistry, biological activity, and chemotherapeutic potential of betulinic acid for the prevention and treatment of cancer and HIV infection. Med Res Rev 24:90-114, 2004.
27. Zheng ZW, Song SZ, Wu YL, Lian LH, Wan Y, Nan JX: Betulinic acid prevention of d-galactosamine/lipopolysaccharide liver toxicity is triggered by activation of Bcl-2 and antioxidant mechanisms. J Pharm Pharmacol 63:572-578, 2011.
28. Silva FS, Oliveira PJ, Duarte MF: Oleanolic, Ursolic, and Betulinic Acids as Food Supplements or Pharmaceutical Agents for Type 2 Diabetes: Promise or Illusion? J Agric Food Chem 64:2991-3008, 2016.
29. Ajala-Lawal RA, Aliyu NO, Ajiboye TO: Betulinic acid improves insulin sensitivity, hyperglycemia, inflammation and oxidative stress in metabolic syndrome rats via PI3K/Akt pathways. Arch Physiol Biochem 126:107-115, 2020.
30. Xie W, Hu W, Huang Z, Li M, Zhang H, Huang X, Yao P: Betulinic acid accelerates diabetic wound healing by modulating hyperglycemia-induced oxidative stress, inflammation and glucose intolerance. Burns Trauma 10:tkac007, 2022.
31. Castillo PM, Jimenez-Ruiz A, Carnerero JM, Prado-Gotor R: Exploring Factors for the Design of Nanoparticles as Drug Delivery Vectors. Chemphyschem 19:2810-2828, 2018.
32. Liu HR, Ahmad N, Lv B, Li C: Advances in production and structural derivatization of the promising molecule ursolic acid. Biotechnol J 16:e2000657, 2021.
33. Nguyen HN, Ullevig SL, Short JD, Wang L, Ahn YJ, Asmis R: Ursolic Acid and Related Analogues: Triterpenoids with Broad Health Benefits. Antioxidants (Basel) 10: 2021.
34. Gupta N: A Review on Recent Developments in the Anticancer Potential of Oleanolic Acid and its Analogs (2017-2020). Mini Rev Med Chem 22:600-616, 2022.
35. Bildziukevich U, Ozdemir Z, Wimmer Z: Recent Achievements in Medicinal and Supramolecular Chemistry of Betulinic Acid and Its Derivatives. Molecules 24: 2019.
36. Shultz MD: Two Decades under the Influence of the Rule of Five and the Changing Properties of Approved Oral Drugs. J Med Chem 62:1701-1714, 2019.
37. Ruiz-Picazo A, Lozoya-Agullo I, Gonzalez-Alvarez I, Bermejo M, Gonzalez-Alvarez M: Effect of excipients on oral absorption process according to the different gastrointestinal segments. Expert Opin Drug Deliv 18:1005-1024, 2021.
38. Mierina I, Vilskersts R, Turks M: Delivery Systems for Birch-bark Triterpenoids and their Derivatives in Anticancer Research. Curr Med Chem 27:1308-1336, 2020.
39. Saneja A, Arora D, Kumar R, Dubey RD, Panda AK, Gupta PN: Therapeutic applications of betulinic acid nanoformulations. Ann N Y Acad Sci 1421:5-18, 2018.
40. Wang HM, Soica CM, Wenz G: A comparison investigation on the solubilization of betulin and betulinic acid in cyclodextrin derivatives. Nat Prod Commun 7:289-291, 2012.
41. Zadeh MT, Khamesipour A, Shafiee AM, Ebrahimi SH, Haji Molla HM, Mosaffa N, Ramezani A: Comparative analysis between four model nanoformulations of amphotericin B-chitosan, amphotericin B-dendrimer, betulinic acid-chitosan and betulinic acid-dendrimer for treatment of Leishmania major: real-time PCR assay plus. Int J Nanomedicine 14:7593-7607, 2019.
42. Kumar P, Gautam AK, Kumar U, Bhadauria AS, Singh AK, Kumar D, Mahata T, Maity B, Bera H, Saha S: Mechanistic exploration of the activities of poly(lactic-co-glycolic acid)-loaded nanoparticles of betulinic acid against hepatocellular carcinoma at cellular and molecular levels. Arch Physiol Biochem 128:836-848, 2022.
43. Kumar P, Singh AK, Raj V, Rai A, Keshari AK, Kumar D, Maity B, Prakash A, Maiti S, Saha S: Poly(lactic-co-glycolic acid)-loaded nanoparticles of betulinic acid for improved treatment of hepatic cancer: characterization, in vitro and in vivo evaluations. Int J Nanomedicine 13:975-990, 2018.
44. Saneja A, Kumar R, Singh A, Dhar DR, Mintoo MJ, Singh G, Mondhe DM, Panda AK, Gupta PN: Development and evaluation of long-circulating nanoparticles loaded with betulinic acid for improved anti-tumor efficacy. Int J Pharm 531:153-166, 2017.
45. Garcia-Pinel B, Porras-Alcala C, Ortega-Rodriguez A, Sarabia F, Prados J, Melguizo C, Lopez-Romero JM: Lipid-Based Nanoparticles: Application and Recent Advances in Cancer Treatment. Nanomaterials (Basel) 9: 2019.
46. Porter CJ, Trevaskis NL, Charman WN: Lipids and lipid-based formulations: optimizing the oral delivery of lipophilic drugs. Nat Rev Drug Discov 6:231-248, 2007.
47. Liu Y, Gao D, Zhang X, Liu Z, Dai K, Ji B, Wang Q, Luo L: Antitumor drug effect of betulinic acid mediated by polyethylene glycol modified liposomes. Mater Sci Eng C Mater Biol Appl 64:124-132, 2016.
48. Cavazos-Garduno A, Ochoa Flores AA, Serrano-Nino JC, Martinez-Sanchez CE, Beristain Cl, Garcia HS: Preparation of betulinic acid nanoemulsions stabilized by omega-3 enriched phosphatidylcholine. Ultrason Sonochem 24:204-213, 2015.
49. Nsairat H, Khater D, Sayed U, Odeh F, Al BA, Alshaer W: Liposomes: structure, composition, types, and clinical applications. Heliyon 8:e09394, 2022.
50. Daraee H, Etemadi A, Kouhi M, Alimirzalu S, Akbarzadeh A: Application of liposomes in medicine and drug delivery. Artif Cells Nanomed Biotechnol 44:381-391, 2016.
51. Dhiman N, Sarvaiya J, Mohindroo P: A drift on liposomes to proliposomes: recent advances and promising approaches. J Liposome Res1-15, 2022.
52. Musther H, Olivares-Morales A, Hatley OJ, Liu B, Rostami HA: Animal versus human oral drug bioavailability: do they correlate? Eur J Pharm Sci 57:280-291, 2014.
53. Manconi M, Caddeo C, Manca ML, Fadda AM: Oral delivery of natural compounds by phospholipid vesicles. Nanomedicine (Lond) 15:1795-1803, 2020.
54. He H, Lu Y, Qi J, Zhu Q, Chen Z, Wu W: Adapting liposomes for oral drug delivery. Acta Pharm Sin B 9:36-48, 2019.
55. Amiri S, Dastghaib S, Ahmadi M, Mehrbod P, Khadem F, Behrouj H, Aghanoori MR, Machaj F, Ghamsari M, Rosik J, Hudecki A, Afkhami A, Hashemi M, Los MJ, Mokarram P, Madrakian T, Ghavami S: Betulin and its derivatives as novel compounds with different pharmacological effects. Biotechnol Adv 2019.
56. McMahon DJ, Oommen BS: Supramolecular structure of the casein micelle. J Dairy Sci 91:1709-1721, 2008.
57. Sadiq U, Gill H, Chandrapala J: Casein Micelles as an Emerging Delivery System for Bioactive Food Components. Foods 10: 2021.
58. Ma F, Wei J, Hao L, Shan Q, Li H, Gao D, Jin Y, Sun P: Bioactive Proteins and their Physiological Functions in Milk. Curr Protein Pept Sci 20:759-765, 2019.
59. Jensen RG: The composition of bovine milk lipids: January 1995 to December 2000. J Dairy Sci 85:295-350, 2002.
60. Singh H, Gallier S: Nature's complex emulsion: The fat globules of milk. Food Hydrocoll 68:81, 2017.
61. Lopez C: Milk fat globules enveloped by their biological membrane: Unique colloidal assemblies with a specific composition and structure. Curr Opin Colloid Interface Sci 16:391-404, 2011.
62. Braun K, Hanewald A, Vilgis TA: Milk Emulsions: Structure and Stability. Foods 8:483, 2019.
63. He S, Tang H, Yi H, Xu W, Ma Y, Wang R: Properties of emulsions from milk fat globule membrane and its components. Int J Food Prop 20:S1342-S1353, 2017.
64. Argov N, Lemay DG, German JB: Milk Fat Globule structure & function; nanosciece comes to milk production. Trends Food Sci Technol 19: 2008.
65. Ye A, Anema SG, Singh H: Changes in the surface protein of the fat globules during homogenization and heat treatment of concentrated milk. J Dairy Res 75:347-353, 2008.
66. Ye A, Cui J, Dalgleish D, Singh H: Effect of homogenization and heat treatment on the behavior of protein and fat globules during gastric digestion of milk. J Dairy Sci 100:36-47, 2017.
67. Kielczewska K, Ambroziak K, Krzykowska D, Aljewicz M: The effect of highpressure homogenisation on the size of milk fat globules and MFGM composition in sweet buttermilk and milk. Int Dairy J 113:104898, 2021.
68. Zhou X, Sala G, Sagis LMC: Bulk and interfacial properties of milk fat emulsions stabilized by whey protein isolate and whey protein aggregates . Food Hydrocolloids 109:106100, 2020.
69. Shi J, Kantoff PW, Wooster R, Farokhzad OC: Cancer nanomedicine: progress, challenges and opportunities. Nat Rev Cancer 17:20-37, 2017.
70. Tang Y, Wang X, Li J, Nie Y, Liao G, Yu Y, Li C: Overcoming the Reticuloendothelial System Barrier to Drug Delivery with a "Don't-Eat-Us" Strategy. ACS Nano 13:13015-13026, 2019.
71. Zhao B, Li X, Kong Y, Wang W, Wen T, Zhang Y, Deng Z, Chen Y, Zheng X: Recent advances in nano-drug delivery systems for synergistic antitumor immunotherapy. Front Bioeng Biotechnol 10:1010724, 2022.
72. Dan N: Core-shell drug carriers: liposomes, polymersomes, and niosomes. Chapter 2 from book: Nanostructures for Drug Delivery. Page 63-105, 2017.
73. Siepmann J, Faham A, Clas SD, Boyd BJ, Jannin V, Bernkop-Schnurch A, Zhao H, Lecommandoux S, Evans JC, Allen C, Merkel OM, Costabile G, Alexander MR, Wildman RD, Roberts CJ, Leroux JC: Lipids and polymers in pharmaceutical technology: Lifelong companions. Int J Pharm 558:128-142, 2019.
74. Lu H, Zhang S, Wang J, Chen Q: A Review on Polymer and Lipid-Based Nanocarriers and Its Application to Nano-Pharmaceutical and Food-Based Systems. Front Nutr 8:783831, 2021.
75. Mehanna MM, Mneimneh AT: Formulation and Applications of Lipid-Based Nanovehicles: Spotlight on Self-emulsifying Systems. Adv Pharm Bull 11:56-67, 2021.
76. Sarah Faulkner. Are nanoparticles overhyped? 2017.
   Ref Type: Online Source: https://www.medicaldesignandoutsourcing.com/nanoparticles-an-industry-buzzword-meets-reality/
77. Patra JK, Das G, Fraceto LF, Campos EVR, Rodriguez-Torres MDP, Acosta-Torres LS, Diaz-Torres LA, Grillo R, Swamy MK, Sharma S, Habtemariam S, Shin HS: Nano based drug delivery systems: recent developments and future prospects. J Nanobiotechnology 16:71, 2018.
78. Müller RH. Nanoparticles - the hype and the decline? Why? 2020.
   Ref Type: Online Source: https://www.controlledreleasesociety.org/news/nanoparticles-hype-and-decline-why
79. De Jong WH, Borm PJ: Drug delivery and nanoparticles:applications and hazards. Int J Nanomedicine 3:133-149, 2008.
80. Ditzinger F, Price DJ, Ilie AR, Kohl NJ, Jankovic S, Tsakiridou G, Aleandri S, Kalantzi L, Holm R, Nair A, Saal C, Griffin B, Kuentz M: Lipophilicity and hydrophobicity considerations in bio-enabling oral formulations approaches - a PEARRL review. J Pharm Pharmacol 71:464-482, 2019.
81. Nitin N, Alshehab M. Encapsulating Molecules in Fat Globules.
   WO2020/227030A1. 2022. 30-4-2020. Ref Type: Patent
82. Alshehab M, Nitin N: Encapsulation and release of curcumin using an intact milk fat globule delivery system. Food Funct 10:7121-7130, 2019.
83. Repasky EA, Bankert RB. Use of Milk Globules as Carriers for Drugs.
   US4994496A. 1991. United States of America. 9-9-1987. Ref Type: Patent
84. Broniatowski M, Flasinski M, Wydro P: Investigation of the interactions of lupane type pentacyclic triterpenes with outer leaflet membrane phospholipids--Langmuir monolayer and synchrotron X-ray scattering study. J Colloid Interface Sci 381:116-124, 2012.
85. Chen Y, Sun R, Wang B: Monolayer behavior of binary systems of betulinic acid and cardiolipin: thermodynamic analyses of Langmuir monolayers and AFM study of Langmuir-Blodgett monolayers. J Colloid Interface Sci 353:294-300, 2011.
86. Abboud R, Charcosset C, Greige-Gerges H: Tetra- and Penta-Cyclic Triterpenes Interaction with Lipid Bilayer Membrane: A Structural Comparative Study. J Membr Biol 249:327-338, 2016.
87. Salim M, Eason T, Boyd BJ: Opportunities for milk and milk-related systems as 'new' low-cost excipient drug delivery materials. Adv Drug Deliv Rev 183:114139, 2022.
88. Singh H: Symposium review: Fat globules in milk and their structural modifications during gastrointestinal digestion. J Dairy Sci 102:2749-2759, 2019.
89. Bach Korsholm KK, Heerup C, Rongaard Stange JT, Geng X, Drachmann N, Nordby P, Bekker JP, Ifaoui I, Mullertz A, Torp SP, Stampe OM, Thymann T: Bovine Milk-Derived Emulsifiers Increase Triglyceride Absorption in Newborn Formula-Fed Pigs. Nutrients 13: 2021.
90. Gazquez A, Sabater-Molina M, Dominguez-Lopez I, Sanchez-Campillo M, Torrento N, Tibau J, Moreno-Munoz JA, Rodriguez-Palmero M, Lopez-Sabater MC, Larque E: Milk fat globule membrane plus milk fat increase docosahexaenoic acid availability in infant formulas. Eur J Nutr Online-ahead-of-print: 2022.
91. He S, Ye A: Formation and gastrointestinal digestion of beta-carotene emulsion stabilized by milk fat globule membrane. J Food Process Eng 42:e13301, 2019.
92. Thum C, Roy NC, Everett DW, McNabb WC: Variation in milk fat globule size and composition: A source of bioactives for human health. Crit Rev Food Sci Nutr 63:87-113, 2023.
93. Wang C, Qiao X, Gao Z, Jiang L, Mu Z: Advancement on Milk Fat Globule Membrane: Separation, Identification, and Functional Properties. Front Nutr 8:807284, 2022.
94. Yao C, Zhao S, Liu L, Liu Z, Chen G: Ultrasonic emulsification: basic characteristics, cavitation, mechanism, devices and application. Front Chem Sci Eng 16:1560-1583, 2022.
95. Dukhin AS, Goetz PJ, Travers B: Use of ultrasound for characterizing dairy products. J Dairy Sci 88:1320-1334, 2005.
96. Chakraborty S, Doktorova M, Molugu TR, Heberle FA, Scott HL, Dzikovski B, Nagao M, Stingaciu LR, Standaert RF, Barrera FN, Katsaras J, Khelashvili G, Brown MF, Ashkar R: How cholesterol stiffens unsaturated lipid membranes. Proc Natl Acad Sci U S A 117:21896-21905, 2020.
97. Castor TP: Phospholipid nanosomes. Curr Drug Deliv 2:329-340, 2005.
98. Dehelean CA, Feflea S, Ganta S, Amiji M: Anti-angiogenic effects of betulinic acid administered in nanoemulsion formulation using chorioallantoic membrane assay. J Biomed Nanotechnol 7:317-324, 2011.
99. Harwansh RK, Mukerjee PK, Biswas S: Nanoemulsion as a novel carrier system for improvement of betulinic acid oral bioavailability and hepatoprotective activity. J Mol Liq 237:361, 2017.
100. Bravo-Alfaro DA, Ochoa-Rodriguez RL, Villasenor-Ortega F, Luna-Barcenas G, Garcia HS: Self-nanoemulsifying drug delivery system (SNEDDS) improves the oral bioavailability of betulinic acid. J Mol Liq 364:119946, 2022.
101. Dutta D, Paul B, Mukherjee B, Mondal L, Sen S, Chowdhury C, Debnath MC: Nanoencapsulated betulinic acid analogue distinctively improves colorectal carcinoma in vitro and in vivo. Sci Rep 9:11506, 2019.
102. Wu ZC, Liu XY, Liu JY, Piao JS, Piao MG: Preparation of Betulinic Acid Galactosylated Chitosan Nanoparticles and Their Effect on Liver Fibrosis. Int J Nanomedicine 17:4195-4210, 2022.
103. Zadeh MT, Shafiee AM, Haji Molla HM, Khamesipour A, Mosaffa N, Ramezani A: Novel Nanosized Chitosan-Betulinic Acid Against Resistant Leishmania Major and First Clinical Observation of such parasite in Kidney. Sci Rep 8:11759, 2018.
104. Sardar A, Gautam S, Sinha S, Rai D, Tripathi AK, Dhaniya G, Mishra PR, Trivedi R: Nanoparticles of naturally occurring PPAR-gamma inhibitor betulinic acid ameliorates bone marrow adiposity and pathological bone loss in ovariectomized rats via Wnt/beta-catenin pathway. Life Sci 309:121020, 2022.
105. Dai L, Liu R, Hu L, Wang J, Si CL: Self-assembled PEG-carboxymethylcellulose nanoparticles/a-cyclodextrin hydrogels for injectable and thermosensitive drug delivery. RSC Adv 7:2905, 2017.
106. Dai L, Cao X, Liu KF, Li CX, Zhang GF, Deng LH, Si CL, He J, Lei JD: Self-assembled targeted folate-conjugated eight-arm-polyethylene glycol-betulinic acid nanoparticles for co-delivery of anticancer drugs. J Mater Chem B 3:3754-3766, 2015.

## Claims

1. Composition comprising a pentacyclic triterpenoid compound, wherein the pentacyclic triterpenoid compound is incorporated into milk fat globules or into lipid vesicles having a membrane, wherein the membrane is a tri-layer comprising
- a phospholipid monolayer; and
- a phospholipid bilayer.

2. Composition comprising a pentacyclic triterpenoid compound according to claim 1, wherein one pentacyclic triterpenoid compound, or a combination of two or more different pentacyclic triterpenoid compounds, are comprised in a plurality of milk fat globules.

3. Composition comprising a pentacyclic triterpenoid compound according to any one of the previous claims, wherein the milk fat globules or lipid vesicles have an average diameter of between 0.1 and 15 µm, preferably between 1 and 10 µm, more preferably between 2 and 6 µm, as determined by laser diffraction.

4. Composition comprising a pentacyclic triterpenoid compound according to any one of the previous claims, wherein the pentacyclic triterpenoid compound is betulinic acid, and/or betulin, and/or lupeol, and/or oleanolic acid, and/or ursolic acid.

5. Composition comprising a pentacyclic triterpenoid compound according to any one of the previous claims, wherein the pentacyclic triterpenoid compound is betulinic acid and/or betulin.

6. Composition comprising a pentacyclic triterpenoid compound according to any one of the previous claims, wherein the pentacyclic triterpenoid compound is betulinic acid.

7. Composition comprising a pentacyclic triterpenoid compound according to any one of the previous claims, wherein the pentacyclic triterpenoid compound is comprised in a liquid emulsion formulation, preferably wherein the liquid emulsion formulation comprises at least 10, 15, 20, 25, 30, 35, 40, 50, 60, 70 mg/ml pentacyclic triterpenoid compound of which preferably at least 50, 60, 70, 80, 90 wt.% is incorporated into the milk fat globules or lipid vesicles.

8. Composition comprising a pentacyclic triterpenoid compound according to any one of claims 1-6, wherein the pentacyclic triterpenoid compound is comprised in a powder formulation which preferably is obtained by spray drying or freeze drying technology applied on the liquid formulation according to claim 7.

9. Composition comprising a pentacyclic triterpenoid compound according to any one of the previous claims, wherein the pentacyclic triterpenoid compound is for oral administration and/or comprised in a food composition, beverage composition, food supplement composition, dietary supplement composition, nutraceutical composition or pharmaceutical composition for oral administration.

10. Composition comprising a pentacyclic triterpenoid compound according to any one of the claims 1 - 8, wherein the pentacyclic triterpenoid compound is for topical administration to the skin preferably as an ointment or cream for medicinal or cosmetic uses.

11. Composition comprising a pentacyclic triterpenoid compound according to any of the previous claims, for therapeutic use.

12. Composition comprising a pentacyclic triterpenoid compound according to any one of the previous claims, for use in the treatment or prevention of cancer, type 2 diabetes mellitus, obesity, metabolic syndrome, cardiovascular disease, skin disorder, (chronic) inflammation, or Parkinson's disease and/or related motor impairment, preferably for use in the treatment or prevention of cancer.

13. Composition comprising a pentacyclic triterpenoid compound according to any one of the previous claims, for use in the treatment or prevention of cancer, wherein the cancer is lung cancer, colorectal cancer, breast cancer, prostate cancer, cervical cancer or melanoma.

14. Method for preparing the composition comprising a pentacyclic triterpenoid compound according to any one of the previous claims, the method comprising the steps of:
a) providing a composition comprising lipid vesicles having a trilayer membrane comprising a monolayer of phospholipids and a bilayer of phospholipids, preferably milk fat globules;
b) providing the pentacyclic triterpenoid composition according to any one of the previous claims by mixing the composition provided in a) with one or more different pentacyclic triterpenoid compounds, preferably by applying high shear mixing and/or sonication and/or high pressure homogenization technique, thereby achieving the incorporation of the triterpenoid compound into the lipid vesicles, preferably milk fat globules, and the assembly of milk fat globules, or lipid vesicles, incorporating the triterpenoid compound.

15. Method according to claim 14, wherein in step b) sonication is applied to agitate the milk fat globules, or lipid vesicles, together with the pentacyclic triterpenoid compound and to thereby effect the dispersion of the pentacyclic triterpenoid compound in an emulsion containing the milk fat globules or lipid vesicles, and to achieve the incorporation of the pentacyclic triterpenoid compound into the milk fat globules or lipid vesicles.

16. Method according to any one of claims 14-15, wherein in step a) or b), milk protein is added, preferably casein protein, casein micelles and/or whey protein.

17. Composition comprising a pentacyclic triterpenoid compound according to any one of claims 1-13 in combination with milk protein, preferably casein protein, casein micelles and/or whey protein.

## Patentansprüche

1. Zusammensetzung, umfassend eine pentacyclische Triterpenoidverbindung, wobei die pentacyclische Triterpenoidverbindung in Milchfettkügelchen oder in Lipidvesikel, die eine Membran aufweisen, eingebaut ist, wobei die Membran eine Dreischicht ist, umfassend
- eine Phospholipid-Monoschicht; und
- eine Phospholipid-Doppelschicht.

2. Zusammensetzung, umfassend eine pentacyclische Triterpenoidverbindung gemäß Anspruch 1, wobei eine pentacyclische Triterpenoidverbindung oder eine Kombination aus zwei oder mehr verschiedenen pentacyclischen Triterpenoidverbindungen in einer Vielzahl von Milchfettkügelchen umfasst sind.

3. Zusammensetzung, umfassend eine pentacyclische Triterpenoidverbindung gemäß einem der vorhergehenden Ansprüche, wobei die Milchfettkügelchen oder Lipidvesikel einen durchschnittlichen Durchmesser zwischen 0,1 und 15 µm, vorzugsweise zwischen 1 und 10 µm, weiter bevorzugt zwischen 2 und 6 µm, wie durch Laserbeugung bestimmt, aufweisen.

4. Zusammensetzung, umfassend eine pentacyclische Triterpenoidverbindung gemäß einem der vorhergehenden Ansprüche, wobei die pentacyclische Triterpenoidverbindung Betulinsäure und/oder Betulin und/oder Lupeol und/oder Oleanolsäure und/oder Ursolsäure ist.

5. Zusammensetzung, umfassend eine pentacyclische Triterpenoidverbindung gemäß einem der vorhergehenden Ansprüche, wobei die pentacyclische Triterpenoidverbindung Betulinsäure und/oder Betulin ist.

6. Zusammensetzung, umfassend eine pentacyclische Triterpenoidverbindung gemäß einem der vorhergehenden Ansprüche, wobei die pentacyclische Triterpenoidverbindung Betulinsäure ist.

7. Zusammensetzung, umfassend eine pentacyclische Triterpenoidverbindung gemäß einem der vorhergehenden Ansprüche, wobei die pentacyclische Triterpenoidverbindung in einer flüssigen Emulsionsformulierung umfasst ist, vorzugsweise wobei die flüssige Emulsionsformulierung mindestens 10, 15, 20, 25, 30, 35, 40, 50, 60, 70 mg/ml der pentacyclischen Triterpenoidverbindung umfasst, von der vorzugsweise mindestens 50, 60, 70, 80, 90 Gew.-% in die Milchfettkügelchen oder Lipidvesikel eingebaut sind.

8. Zusammensetzung, umfassend eine pentacyclische Triterpenoidverbindung gemäß einem der Ansprüche 1-6, wobei die pentacyclische Triterpenoidverbindung in einer Pulverformulierung umfasst ist, die vorzugsweise durch Sprühtrocknungs- oder Gefriertrocknungstechnologie erhalten wird, die auf die flüssige Formulierung gemäß Anspruch 7 angewendet wird.

9. Zusammensetzung, umfassend eine pentacyclische Triterpenoidverbindung gemäß einem der vorhergehenden Ansprüche, wobei die pentacyclische Triterpenoidverbindung zur oralen Verabreichung bestimmt ist und/oder in einer Lebensmittelzusammensetzung, Getränkezusammensetzung, Nahrungsergänzungsmittelzusammensetzung, diätetischen Ergänzungsmittelzusammensetzung, nutrazeutischen Zusammensetzung oder pharmazeutischen Zusammensetzung zur oralen Verabreichung umfasst ist.

10. Zusammensetzung, umfassend eine pentacyclische Triterpenoidverbindung gemäß einem der Ansprüche 1-8, wobei die pentacyclische Triterpenoidverbindung zur topischen Verabreichung auf die Haut bestimmt ist, vorzugsweise als Salbe oder Creme für medizinische oder kosmetische Verwendungen.

11. Zusammensetzung, umfassend eine pentacyclische Triterpenoidverbindung gemäß einem der vorhergehenden Ansprüche, zur therapeutischen Verwendung.

12. Zusammensetzung, umfassend eine pentacyclische Triterpenoidverbindung gemäß einem der vorhergehenden Ansprüche, zur Verwendung bei der Behandlung oder Vorbeugung von Krebs, Typ-2-Diabetes mellitus, Adipositas, metabolischem Syndrom, Herzkreislauferkrankungen, Hauterkrankungen, (chronischen) Entzündungen oder der Parkinson-Krankheit und/oder damit verbundenen motorischen Beeinträchtigungen, vorzugsweise zur Verwendung bei der Behandlung oder Vorbeugung von Krebs.

13. Zusammensetzung, die eine pentacyclische Triterpenoidverbindung gemäß einem der vorhergehenden Ansprüche, zur Verwendung bei der Behandlung oder Vorbeugung von Krebs, wobei der Krebs Lungenkrebs, kolorektaler Krebs, Brustkrebs, Prostatakrebs, Gebärmutterhalskrebs oder Melanom ist.

14. Verfahren zur Herstellung der Zusammensetzung, umfassend eine pentacyclische Triterpenoidverbindung gemäß einem der vorhergehenden Ansprüche, wobei das Verfahren die folgenden Schritte umfasst:
a) Bereitstellen einer Zusammensetzung, die Lipidvesikel mit einer Dreischichtmembran umfasst, die eine Monoschicht aus Phospholipiden und eine Doppelschicht aus Phospholipiden, vorzugsweise Milchfettkügelchen, umfasst;
b) Bereitstellen der pentacyclischen Triterpenoid-Zusammensetzung gemäß einem der vorhergehenden Ansprüche durch Mischen der in a) bereitgestellten Zusammensetzung mit einer oder mehreren unterschiedlichen pentacyclischen Triterpenoidverbindungen, vorzugsweise durch Anwenden einer Hochscher-Misch- und/oder Sonikations- und/oder Hochdruckhomogenisierungstechnik, wodurch der Einbau der Triterpenoidverbindung in die Lipidvesikel, vorzugsweise Milchfettkügelchen, und die Zusammenfügung von Milchfettkügelchen oder Lipidvesikeln, die die Triterpenoidverbindung einbauen, erreicht wird.

15. Verfahren gemäß Anspruch 14, wobei in Schritt b) Sonikation angewendet wird, um die Milchfettkügelchen oder Lipidvesikel zusammen mit der pentacyclischen Triterpenoidverbindung zu bewegen und dadurch die Dispersion der pentacyclischen Triterpenoidverbindung in einer Emulsion, die die Milchfettkügelchen oder Lipidvesikel enthält, zu bewirken und den Einbau der pentacyclischen Triterpenoidverbindung in die Milchfettkügelchen oder Lipidvesikel zu erreichen.

16. Verfahren gemäß einem der Ansprüche 14-15, wobei in Schritt a) oder b) Milchprotein zugesetzt wird, vorzugsweise Caseinprotein, Caseinmizellen und/oder Molkenprotein.

17. Zusammensetzung, umfassend eine pentacyclische Triterpenoidverbindung gemäß einem der Ansprüche 1-13 in Kombination mit Milchprotein, vorzugsweise Caseinprotein, Caseinmizellen und/oder Molkenprotein.

## Revendications

1. Composition comprenant un composé triterpénoïde pentacyclique, dans laquelle le composé triterpénoïde pentacyclique est incorporé dans des globules gras du lait ou dans des vésicules lipidiques ayant une membrane, dans laquelle la membrane est une triple couche comprenant
- une monocouche de phospholipides ; et
- une bicouche de phospholipides.

2. Composition comprenant un composé triterpénoïde pentacyclique selon la revendication 1, dans laquelle un composé triterpénoïde pentacyclique, ou une combinaison de deux composés triterpénoïdes pentacycliques différents ou plus, sont compris dans une pluralité de globules gras du lait.

3. Composition comprenant un composé triterpénoïde pentacyclique selon l'une quelconque des revendications précédentes, dans laquelle les globules gras du lait ou les vésicules lipidiques ont un diamètre moyen compris entre 0,1 et 15 µm, de préférence entre 1 et 10 µm, plus préférablement entre 2 et 6 µm, tel que déterminé par diffraction laser.

4. Composition comprenant un composé triterpénoïde pentacyclique selon l'une quelconque des revendications précédentes, dans laquelle le composé triterpénoïde pentacyclique est l'acide bétulinique, et/ou la bétuline, et/ou le lupéol, et/ou l'acide oléanolique, et/ou l'acide ursolique.

5. Composition comprenant un composé triterpénoïde pentacyclique selon l'une quelconque des revendications précédentes, dans laquelle le composé triterpénoïde pentacyclique est l'acide bétulinique et/ou la bétuline.

6. Composition comprenant un composé triterpénoïde pentacyclique selon l'une quelconque des revendications précédentes, dans laquelle le composé triterpénoïde pentacyclique est l'acide bétulinique.

7. Composition comprenant un composé triterpénoïde pentacyclique selon l'une quelconque des revendications précédentes, dans laquelle le composé triterpénoïde pentacyclique est compris dans une formulation d'émulsion liquide, de préférence dans laquelle la formulation d'émulsion liquide comprend au moins 10, 15, 20, 25, 30, 35, 40, 50, 60, 70 mg/ml de composé triterpénoïde pentacyclique dont de préférence au moins 50, 60, 70, 80, 90 % en poids sont incorporés dans les globules gras du lait ou les vésicules lipidiques.

8. Composition comprenant un composé triterpénoïde pentacyclique selon l'une quelconque des revendications 1 à 6, dans laquelle le composé triterpénoïde pentacyclique est compris dans une formulation en poudre qui est de préférence obtenue par une technologie de séchage par pulvérisation ou de lyophilisation appliquée à la formulation liquide selon la revendication 7.

9. Composition comprenant un composé triterpénoïde pentacyclique selon l'une quelconque des revendications précédentes, dans laquelle le composé triterpénoïde pentacyclique est destiné à l'administration orale et/ou compris dans une composition d'aliment, une composition de boisson, une composition de complément alimentaire, une composition de supplément diététique, une composition nutraceutique ou une composition pharmaceutique pour une administration orale.

10. Composition comprenant un composé triterpénoïde pentacyclique selon l'une quelconque des revendications 1 à 8, dans laquelle le composé triterpénoïde pentacyclique est pour une administration topique sur la peau de préférence sous forme de pommade ou de crème pour des utilisations médicinales ou cosmétiques.

11. Composition comprenant un composé triterpénoïde pentacyclique selon l'une quelconque des revendications précédentes, pour une utilisation thérapeutique.

12. Composition comprenant un composé triterpénoïde pentacyclique selon l'une quelconque des revendications précédentes, pour une utilisation dans le traitement ou la prévention de cancer, diabète sucré de type 2, obésité, syndrome métabolique, maladie cardiovasculaire, trouble cutané, inflammation (chronique), ou maladie de Parkinson et/ou déficience motrice associée, de préférence pour une utilisation dans le traitement ou la prévention d'un cancer.

13. Composition comprenant un composé triterpénoïde pentacyclique selon l'une quelconque des revendications précédentes, pour une utilisation dans le traitement ou la prévention d'un cancer, le cancer étant un cancer du poumon, un cancer colorectal, un cancer du sein, un cancer de la prostate, un cancer du col de l'utérus ou un mélanome.

14. Procédé pour la préparation de la composition comprenant un composé triterpénoïde pentacyclique selon l'une quelconque des revendications précédentes, le procédé comprenant les étapes consistant à :
a) fournir une composition comprenant des vésicules lipidiques ayant une membrane à triple couche comprenant une monocouche de phospholipides et une bicouche de phospholipides, de préférence des globules gras du lait ;
b) fournir la composition de triterpénoïde pentacyclique selon l'une quelconque des revendications précédentes en mélangeant la composition fournie en a) avec un ou plusieurs composés triterpénoïdes pentacycliques différents, de préférence en appliquant une technique de mélange à cisaillement élevé et/ou de sonication et/ou d'homogénéisation à haute pression, réalisant de ce fait l'incorporation du composé triterpénoïde dans les vésicules lipidiques, de préférence les globules gras du lait, et l'assemblage de globules gras du lait, ou de vésicules lipidiques, incorporant le composé triterpénoïde.

15. Procédé selon la revendication 14, dans lequel à l'étape b) la sonication est appliquée pour agiter les globules gras du lait, ou les vésicules lipidiques, conjointement avec le composé triterpénoïde pentacyclique et pour de ce fait effectuer la dispersion du composé triterpénoïde pentacyclique dans une émulsion contenant les globules gras du lait ou les vésicules lipidiques, et pour réaliser l'incorporation du composé triterpénoïde pentacyclique dans les globules gras du lait ou les vésicules lipidiques.

16. Procédé selon l'une quelconque des revendications 14 à 15, dans lequel à l'étape a) ou b), de la protéine de lait est ajoutée, de préférence de la protéine de caséine, des micelles de caséine et/ou de la protéine de lactosérum.

17. Composition comprenant un composé triterpénoïde pentacyclique selon l'une quelconque des revendications 1 à 13 en combinaison avec des protéines du lait, de préférence des protéines de caséine, des micelles de caséine et/ou des protéines de lactosérum.
